# EUROPEAN PATENT SPECIFICATION

(11) **EP 2 908 772 B1**
(45) Date of publication and mention of the grant of the patent: **11.12.2019**
(21) Application number: 13849726.8
(22) Date of filing: 22.10.2013
(51) Int. Cl.: A61F 2/915

(54) **MEDICAL DEVICE FOR IMPLANTATION INTO LUMINAL STRUCTURES**
MEDIZINISCHE VORRICHTUNG ZUR IMPLANTATION IN LUMINALE STRUKTUREN
DISPOSITIF MÉDICAL À IMPLANTER DANS DES STRUCTURES LUMINALES

(30) Priority: 22.10.2012 US 201261716660 P
(43) Date of publication of application: 26.08.2015
(73) Proprietor: ORBUSNEICH MEDICAL PTE. LTD, 079906 Singapore (SG)
(72) Inventor: COTTONE, Robert, J., Davie, FL 33330 (US); JUMAN, Muhammad, Ike, Davie, FL 33331 (US); PAZIENZA, John, Pompano Beach, FL 33060 (US); IPPOLITO, Jennifer, Plantation, FL 33322 (US)
(74) Representative: Jilderda, Anne Ayolt
(86) International application number: PCT/US2013/066128
(87) International publication number: WO 2014/066356

(56) References cited:
- EP-B1- 1 341 482
- WO-A1-2012/103527
- WO-A1-2013/134560
- US-A1- 2006 276 880
- US-A1- 2008 294 239
- US-A1- 2009 240 317
- US-A1- 2009 240 317
- US-A1- 2010 131 044
- US-A1- 2011 029 064
- US-A1- 2011 238 156
- US-A1- 2011 313 508
- US-B2- 7 175 654

## Description

### Field of the Invention

The present invention relates to a scaffold of a bio-degradable composition, comprising a primarily cylindrically shaped main body with a cylindrical axis having a plurality of circumferential elements that circumscribe a circumference of said cylindrical main body, wherein a circumferential element is connected to an adjacent circumferential element by a plurality of first connection elements to form a pair of circumferential elements, wherein a pair of circumferential elements is connected to an adjacent pair of circumferential elements by a plurality of second connection elements, wherein the circumferential elements comprise a plurality of undulations, having alternating peaks and valleys, and wherein first and second connection elements are connected with one another to form at least one contiguous spiral pattern, when the scaffold is expanded, and specifically to such stents. In particular, the present invention relates to geometric designs of stents which exhibit a high degree of radial strength and flexibility and which can be formed from bioabsorbable polymers.

### Background of the Invention

Stents are vascular scaffolds that are positioned in diseased vessel segments to support the vessel walls. During angioplasty, stents are used to repair and reconstruct blood vessels. Placement of a stent in the affected arterial segment prevents elastic recoil and closing of the artery. Stents also prevent local dissection of the artery along the medial layer. Physiologically, stents may be placed inside the lumen of any space, such as an artery, vein, bile duct, urinary tract, alimentary tract, tracheobronchial tree, cerebral aqueduct or genitourinary system. Stents may also be placed inside the lumen of non-human animals, such as primates, horses, cows, pigs and sheep.

In general, there are two types of vascular scaffolds or stents: self-expanding and balloon-expandable. Self-expanding stents automatically expand once they are released and assume a deployed, expanded state. A self-expanding stent is placed in the vessel by inserting the stent in a compressed state into the affected region, e.g., an area of stenosis. Compression or crimping of the stent can be achieved using crimping equipment (see, http://www.machinesolutions.org/stent_crimping.htm, April, 2009). The stent may also be compressed using a tube that has a smaller outside diameter than the inner diameter of the affected vessel region. Once the compressive force is removed or the temperature raised, the stent expands to fill the lumen of the vessel. When the stent is released from confinement in the tube, the stent expands to resume its original shape, in the process becoming securely fixed inside the vessel against the wall.

A balloon-expandable stent is expanded using an inflatable balloon catheter. Balloon-expandable stents may be implanted by mounting the stent in an unexpanded or crimped state on a balloon segment of a catheter. The catheter, after having the crimped stent placed on it, is inserted through a puncture in a vessel wall and moved through the vessel until it is positioned in the portion of the vessel that is in need of repair. The stent is then expanded by inflating the balloon catheter against the inside wall of the vessel. Specifically, the stent is plastically deformed by inflating the balloon so that the diameter of the stent is increased and the stent expanded.

There are functional limitations common to many stents. These include, for example, comparative rigidity of the stent in a crimped as well as deployed state, and limited flexibility making delivery and placement in narrow vessels difficult. The present invention provides a geometric design for a stent that offers both a high degree of flexibility and significant radial strength. The stent may also be formed from bioabsorbable polymers. The design of this stent also allows it to be inserted into small diameter vessels having tortuous vascular anatomy. US2006276880 A1 relates to a radially expandable vessel support which comprises a multitude of zigzag shaped annular elements, interconnected in a flexible way by bending elements.

### Summary of the Invention

The invention is defined by claim 1 and according to the invention a scaffold of the type described in the opening paragraph is characterized in that said first connections elements are S-shaped or Z-shaped and connect a valley of the undulations in one circumferential element within a pair to a peak of the undulations in the other circumferential element of that pair, and in that said second connection elements are S-shaped or Z-shaped and connect a peak of the undulations in one circumferential element within a pair to a valley of the undulations in an adjacent circumferential element of an adjacent pair of circumferential elements. The present scaffold comprises a plurality of circumferential elements, where at least two circumferential elements are connected by a plurality of first connection elements to form a pair of circumferential elements, one pair of circumferential elements is connected to an adjacent pair of circumferential elements by a plurality of second connection elements. When the scaffold is expanded, at least a portion of the first and/or second connection elements form at least one contiguous spiral pattern. The spiral pattern comprises at least one first connection element and at least one second connection element.

The circumferential elements can be comprised of a plurality of undulations. In certain embodiments, the undulations form a repeating or non-repeating sinusoidal pattern. There may be at least two first connection elements connecting the pair of circumferential elements. In one embodiment, there are three first connection elements connecting the pair of circumferential elements. In another embodiment, there are three second connection elements connecting adjacent pairs of circumferential elements.

The sinusoidal pattern of one circumferential element in the pair may be 180° degrees out-of-phase with the sinusoidal pattern of the second circumferential element in the pair. Each peak of the undulation in one circumferential element of the pair may be connected by the first connection element with each valley in the second circumferential element in the pair. The sinusoidal patterns of one pair of circumferential elements may be in-phase with the sinusoidal pattern of an adjacent pair of circumferential elements. Every other peak of the undulation in one circumferential element in one pair may be connected by the second connection element to the valley of the corresponding undulation in the circumferential element in the adjacent pair of circumferential elements.

The first connection element is S-shaped or Z-shaped. It may contain a marker dot.

The second connection element is S-shaped or Z-shaped. It may contain a marker dot. The length of the second connection element can be greater than, equal to or less than the length of the first connection element.

When expanded, the spiral pattern can form a helix. If there are two helices, they may or may not be substantially parallel to each other. In one embodiment, there are two or more helices equidistant from the cylindrical axis of the scaffold. The spiral pattern can be formed from a repeated unit comprising the first connection element and second connection element.

The scaffold may comprise bioabsorbable polymeric material, a biocorrodable metal or combinations thereof. Non-limiting examples of bioabsorbable polymeric material include poly-L-lactide (PLLA). Non-limiting examples of biocorrodable metals including iron and magnesium. The scaffold can be in an expanded, partially expanded, unexpanded, or as manufactured state.

### Brief Description of the Drawings

Figure 1 shows a cut pattern of the scaffold.
Figure 2A shows a cut pattern of the scaffold with the circumferential elements having varying lengths.
Figure 2B shows the varying amplitude of circumferential elements.
Figure 2C shows a repeating sinusoid pattern.
Figure 2D shows a non-repeating sinusoid pattern.
Figure 3 shows a detailed view of adjacent circumferential elements.
Figure 4A shows the details of the connection elements at one end of the scaffold.
Figure 4B illustrates various embodiments of the various connection elements.
Figure 4C shows a perspective view of a marker dot.
Figure 4D shows cross sections of various embodiments of marker dots.
Figures 5A-5C show three dimensional views of the scaffold from various angles.
Figure 6 shows a cut pattern of another embodiment of the scaffold.
Figure 7 shows a cut pattern of the scaffold with an alternative spiral pattern.
Figure 8 illustrates a cut pattern of the scaffold containing a variety of connection elements with different shapes.
Figure 9 shows a cut pattern of the scaffold with an alternative spiral pattern together with circumferential elements of varying length.
Figures 10A and 10B show side views of a three-dimensional perspective of the scaffold in a crimped state.
Figures 11A and 11B illustrate cut patterns of the present scaffolds with alternative spiral patterns.
Figure 12 illustrates the functionality of the connection element.
Figure 13 illustrates the geometry of the scaffold and connection elements (first and second connection elements) on expansion.
Figures 14A and 14B illustrate the geometry of the connection elements.
Figure 14C shows various embodiments of how the connection elements can be attached to adjacent circumferential elements.
Figures 15A-E illustrates where the connection elements can attach along the undulations.
Figure 16 shows the radial displacement of the attachment of connection element to adjacent undulations.
Figures 17A and 17B show an example of phasing in adjacent circumferential elements.
Figure 18 shows a cut pattern of another embodiment of the scaffold where there are three first connection elements between two circumferential elements.
Figure 19A shows a cut pattern of another embodiment of the scaffold. In this embodiment, there are three first connection elements between two circumferential elements, and the circumferential elements are comprised of a plurality of linear segments.
Figures 19B - 19E show detailed views of parts of the scaffold in Figure 19A.
Figure 19F shows the cross section of the marker dot in Figure 19A.
Figure 19G shows a side view of the scaffold in Figure 19A.

### Detailed Description of the Invention

The present invention relates to expandable vascular scaffolds including stents. The overall design of the scaffold is based on a modular design that comprises pairs of circumferential elements connected by one or more connection elements (the terms connecting and connection are used interchangeably here). Using a modular approach, the scaffold can be assembled from circumferential elements and connection elements that vary in length and design. When expanded, the connection elements form a spiral pattern, which can be a helix.

The vascular scaffolds may be formed from a bioabsorbable polymer, a biocorrodable metal, or combinations thereof. Non-limiting examples of bioabsorbable polymers include poly-L-lactide (PLLA), poly-D-lactide (PDLA), poly(D,L-lactide) (PDLLA), poly(desaminotyrosil-tyrosine ethyl ester) carbonate, poly(caprolactone) (PCL), and poly(anhydride ester) salicylic acid. Non-limiting examples of biocorrodable metal include iron, magnesium, and magnesium alloy. The scaffolds may also be formed from various combinations of metals and polymers. U.S. Patent Nos. 7,846,361; 7,897,224 and 8,137,603. U.S. Patent Publication No. 2010/0093946. Alexy, et al., BioMed Research International, 2013, Article ID 137985.

Generally, the scaffold is a cylindrical object having a cylindrical axis running the length of the cylinder. The modular geometric design of the present invention exhibits a high degree of flexibility and significant radial strength. Generally, the scaffolds have a primarily cylindrical shaped main body that has a plurality of expandable circumferential elements. The circumferential elements can vary in length. At least two circumferential elements may be connected to form a pair of circumferential elements. There may be one or more connection elements between the circumferential elements forming the pair of circumferential elements. The connection elements may be found in a variety of different geometric shapes, including linear, curvilinear or combinations of the two shapes. Each pair of circumferential elements is connected to an adjacent pair of circumferential elements by at least one connection element. The number of connection elements between circumferential elements in a pair or between pairs of circumferential elements can vary.

As used herein, the term "circumferential elements" refers to structural elements circumscribing the circumference of the present scaffold which may be in the form of a cylinder. In one embodiment, the circumferential element is bounded by two hypothetical planes which are substantially perpendicular to the cylindrical axis of the scaffold. A circumferential element may comprise (or consist of) a plurality of undulations.

When the vascular scaffold is expanded, at least a portion of the connection elements connecting the circumferential elements in the pairs and/or the connection elements connecting the circumferential elements in adjacent pairs form a contiguous spiral pattern.

In one embodiment, the contiguous spiral pattern is oriented substantially parallel to the cylindrical axis of the scaffold. The contiguous spiral pattern may also take other orientations. The contiguous spiral pattern may form a helix and there may be one or more helices in a particular embodiment, e.g., double or triple helix, or 4, 5, 6 or higher numbers of helices. When there is more than one helix, adjacent helices may be substantially parallel to each other. Adjacent helices may not be parallel to each other. In one embodiment, there are two or more helices equidistant from the cylindrical axis of the scaffold.

The circumferential elements may be uniform in shape. Alternatively a circumferential element may be comprised of a variety of different shapes. For example, the circumferential elements may be formed from a series of undulations which may be in a sinusoidal pattern, a sawtooth pattern, a square wave pattern or any other type of repeating or non-repeating pattern, e.g., a combination of sinusoidal and sawtooth. The amplitude of the undulations may vary within one circumferential element or between two circumferential elements (amplitude is the peak deviation of the function from zero). The amplitude and frequency of the undulations can also vary. For example, a circumferential element can be comprised of a sinusoidal pattern having a repeated pattern of varying amplitudes, 2:1:2:1, 2:1, etc., where, the ratio of the amplitudes of the undulations are represented by the ratio shown. Other ratios are also possible, 3:1, 4:1, 5:1, etc. The circumferential elements may be comprised of one or more segments with each segment having its own undulation pattern. The number of segments in each circumferential element may vary from 1 to N, e.g., 1, 2, 3, 4, 5, 6, 7, 8, 9 or 10. Thus, the circumferential element can be assembled in a modular fashion from various segments which may be alike or different. In the scaffold, the length of all the circumferential elements may be the same. Alternatively, the length of the circumferential elements may vary, e.g., in several different ways. For example, the length of the circumferential elements within one pair may be the same, while the length of the circumferential elements closer to one end of the scaffold may be greater than or less than the length of the circumferential elements closer to the middle of the scaffold.

The number of connection elements connecting adjacent circumferential elements (first connection element, or second connection element) can range from 1 to N, e.g., 1, 2, 3, 4, 5, 6, 7, 8, 9, 10 or higher numbers, 10-20. The shape of the connecting elements may be linear, curvilinear, S-shaped, reverse S-shaped, Z-shaped, reverse Z-shaped, or any other combination of shapes, including, for example, a linear and curvilinear section. Similarly, the number of connecting elements connecting adjacent pairs of circumferential elements may range from 1 to N (e.g., 1, 2, 3,4, 5, 6, 7, 8, 9 or 10) and the shape of such connecting elements may be linear, curvilinear, S-shaped, reverse S-shaped, Z-shaped, reverse Z-shaped, or any combination thereof. The connecting elements may assume a variety of angles relative to the cylindrical axis of the scaffold, including, 0-20°, 20-40°, 40-60° or 60-80°; furthermore, the angle of these connecting elements may be positive or negative relative to the cylindrical axis of the scaffold. If the connecting elements are curvilinear, they may be concave and convex with the curvature present at selected portions of the connecting elements; the degree of curvature may also vary within one connecting element. The number of connecting elements can be adapted to modify the flexibility of the scaffold with decreasing flexibility generally being present as the number of connecting elements increases.

When the connection element is S-shaped, it may have a substantially S-shaped structure. In one embodiment, the S-shaped connection elements have a double curved structure which allows for more slack between circumferential elements, enabling greater expansion of the scaffold. The longer this S-shaped segment, the more slack and expandability there is in the structure. An S-shaped connection element may be smooth or may be angular. In another embodiment, the S-shaped connection element includes at least three substantially linear portions: a first linear portion being substantially parallel to the cylindrical axis of the scaffold (e.g., forming an angle between about 0 degree and about 20 degrees with respect to the cylindrical axis of the scaffold); a second linear portion being substantially perpendicular to the axis (e.g., forming an angle between about 70 degree and about 90 degrees with respect to the cylindrical axis of the scaffold); and a third linear portion being substantially parallel to the axis (e.g., forming an angle between about 0 degree and about 20 degrees with respect to the cylindrical axis of the scaffold). In still another embodiment, the S-shaped connection element includes at least three substantially linear portions: a first linear portion being substantially parallel to the cylindrical axis of the scaffold (e.g., forming an angle between about 0 degree and about 20 degrees with respect to the cylindrical axis of the scaffold); a second linear portion being substantially perpendicular to the first linear portion (e.g., the second linear portion forming an angle between about 70 degree and about 90 degrees with respect to the first linear portion); and a third linear portion being substantially parallel to the first linear portion (e.g., the third linear portion forming an angle between about 0 degree and about 20 degrees with respect to the first linear portion) or perpendicular to the second linear portion (e.g., the third linear portion forming an angle between about 70 degree and about 90 degrees with respect to the second linear portion).

When the connection element is Z-shaped, it has a substantially Z-shaped structure.

When there is more than one connection element between adjacent circumferential elements, the connection elements are positioned symmetrically or asymmetrically at radial positions along the circumference of the scaffold. If the connection elements are positioned symmetrically, the radial distance between each pair of connection elements, e.g., A-B and B-C, is equal.

The radial positions listed for the connection elements here are only provided for illustration purposes and the connection elements may be positioned by one of ordinary skill in the art without undue experimentation at any point along the circumference of the scaffold with respect to the cylindrical axis. For example, the positioning of the connection elements may be determined by dividing 360° by n where n is the number of connection elements between adjacent circumferential elements. Where n = 3, the connection elements may be positioned symmetrically at approximately 120° intervals around the circumference of the stent. When there are two equally spaced connection elements between adjacent circumferential elements, they are situated approximately 180° with respect to one another. In other words, the two connection elements are oppositely oriented with respect to one another.

For the purposes of reference only, the connection elements connecting the circumferential elements in each pair are referred to as first connection elements, while connection elements connecting the circumferential elements in adjacent pairs of circumferential elements are referred to as second connection elements. The first and second connection elements may be of the same shape or have different shapes. In addition, the shape of the first connection elements connecting the circumferential elements in a pair of circumferential elements may be the same or may very in both shape and length. Similarly, the connection elements connecting adjacent pairs of circumferential elements may be the same or may vary in shape and length. As discussed further below, the first and second connection elements may be configured to allow the vascular scaffold to expand without causing the circumferential elements forming the pairs to significantly bend out of the plane formed by the circumferential element after expansion. Thus, the connection elements between adjacent pairs of circumferential elements (e.g., the second connection elements) may be able to elongate in response to expansion of the scaffold. In one embodiment, these connection elements have an S-shape or are curvilinear.

When the scaffold is expanded, at least a portion of the connection elements connecting the circumferential elements in the pairs and/or the connection elements connecting the circumferential elements in adjacent pairs form a contiguous spiral pattern. In one embodiment, the spiral pattern comprises at least a portion of the first and second connection elements. In another embodiment, the spiral pattern comprises at least a portion of the first connection elements. In a third embodiment, the spiral pattern comprises at least a portion of the second connection elements. In a fourth embodiment, the spiral pattern comprises at least a portion of the first and second connection elements, and at least a portion of the circumferential elements. In a fifth embodiment, the spiral pattern comprises at least a portion of the first connection elements, and at least a portion of the circumferential elements. In a sixth embodiment, the spiral pattern comprises at least a portion of the second connection elements, and at least a portion of the circumferential elements.

The length of a connection element refers to the absolute distance of travel along the connection element starting from one end of the connection element traveling along the distance to the other end of the connection element.

The length of the second connection element can be greater than, equal to or less than the length of the first connection element.

The undulations of the circumferential elements can form peaks and valleys with respect to either proximal or distal end of the vascular scaffold. The first connection elements can connect the circumferential elements in the pair from peak to peak, peak to valley, or valley to valley. Similarly, the second connection elements can connect the circumferential elements between adjacent pairs from peak to peak, peak to valley, or valley to valley. The peak to peak, peak to valley, or valley to valley connections may be between circumferential elements that are in the same cylindrical axial line or shifted by 180° degrees; other shifts, include, but are not limited to, 5°, 60°, 90° and 120°degrees from the same cylindrical axial line. The connection elements may connect any points on adjacent circumferential elements, including, but not limited to, peak, valley, any point on the ascending portion or descending portion of an undulation.

The undulations of one circumferential element in a pair may either be in phase or out of phase with the undulations of the other circumferential element in the pair. If the two circumferential elements are out of phase, the degree of phase difference may range from greater than 0° to 180° degrees, including, but not limited to, 5°, 60°, 90° and 120°degrees.

Similarly, the undulations of one pair may either be in phase or alternatively out of phase with the undulations of an adjacent pair. If the two circumferential elements are out of phase the degree of phase difference may range from greater than 0° to 180° degrees, including, but not limited to, 5°, 60°, 90° and 120°degrees.

The undulations of adjacent circumferential elements may either be in phase or out of phase. If the two circumferential elements are out of phase, the degree of phase difference may range from greater than 0° to 180° degrees, including, but not limited to, 5°, 60°, 90°, 120 and 180°degrees.

When a radial expanding force is applied to the scaffold, such as through an expandable balloon, the circumferential elements expand radially and elongate circumferentially. Conversely, when an external compressive force is exerted on the scaffold, the circumferential elements contract radially and shorten circumferentially. When a radial expanding force is applied to the scaffold, the undulations decrease in amplitude. Conversely, when an external compressive force is exerted on the scaffold, the undulation increases in amplitude.

In another embodiment, the scaffold comprises a plurality of polygons. The polygon has n-sides where n is any positive integers. For example, the polygons may have sides ranging from 3 to 30 (higher order polygons are also encompassed by the designs of the present invention), e.g., 3, 4, 5, 6, 7, 8, 9, 10, 11, 12, 13, 14, 15, 16, 17, 18, 19, 20, 21, 22, 23, 24, 25, 26, 27, 28, 29 and 30 sided polygons, up to an n-sided polygon. The sides of the polygons may be equal or unequal. The opposite sides in a polygon may be substantially parallel to each other when the scaffold is crimped. Opposite sides in a polygon may also take other configurations in relation to each other.

The polygon may be formed from a plurality of undulations which are connected by a plurality of connection elements. For example, the polygon may be a hexagon formed from two undulations connected by two connection elements; a hexagon may comprise a first undulation and a second undulation, which are connected by a first segment and a second segment. The filaments comprising the first and second undulations in each hexagon may have different or identical width, length and thickness. The polygon may also be formed from a plurality of undulations without connection elements. For example, the polygons may be tetragons consisting of two undulations. In higher-order polygons, e.g., n=8-30, the undulations may be connected by a plurality of connection elements.

An undulation may comprise one segment or at least two segments. The segments may be linear or curvilinear. When a segment is curvilinear, the degree of curvature may vary. A segment may be concave or convex. A segment may contain solely linear portions joined together, or solely curved portions joined together. Alternatively, a segment may contain both linear portions and curved portions that are joined together. The segment may comprise at least one bend placed at selected points along its length. For example, a segment may take the shape of a stylized n, C, U, V, etc. A segment may also be in the shape of a loop where the loop may be circular or semicircular. The segment can essentially assume any suitable configuration. The length, width and thickness of the segments of the undulations may be equal or unequal. The two undulations of each polygon across each circumferential component may be identical or may vary. A wide variety of different configurations for the polygons as well as the various segments representing the sides of the polygon are encompassed by the present invention. For example, the segments representing the sides of the polygon may be linear or curvilinear. In one polygon, the length of the segments comprising one undulation may be equal to or greater than the length of the segments of the opposing undulation. The polygon may be convex (i.e., all its interior angles are less than 180°) or non-convex (i.e., it contains at least one interior angle greater than 180°). The polygons can form a continuous, interconnected structure across the body of the scaffold. A circumferential element (or a pair of circumferential elements) may contain different or substantially identical polygons. The polygons of different circumferential element may be different or substantially identical. The surface area of adjacent polygons may be equal or unequal. The surface area of the polygons, i.e., the area encompassed by the sides, can be calculated mathematically from the length of the sides of the polygon. http://mathworld.wolfram.com/PolygonArea.html, April, 2009.

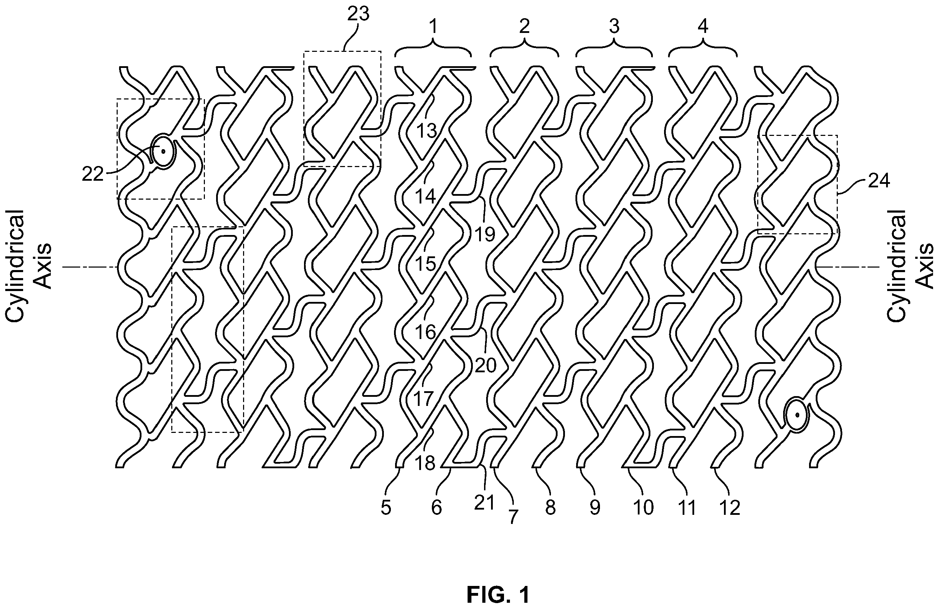

One embodiment of the scaffold of the present invention is illustrated in Figure 1. The pairs of circumferential elements are shown as 1-4. The circumferential elements in the scaffold for pairs 1-4 are labeled in parentheses, 1(5, 6), 2 (7, 8), 3(9, 10) and 4 (11, 12). The connection elements (first connection elements) between two circumferential elements in one pair, 1, are shown as 13-18, while the connection elements between adjacent pairs of circumferential elements 1 and 2 (second connection elements) are shown as 19-21. The scaffold may contain a marker dot, 22. Polygons formed by the undulations and the connection elements of the scaffold are illustrated by the boxes at 23 and 24.

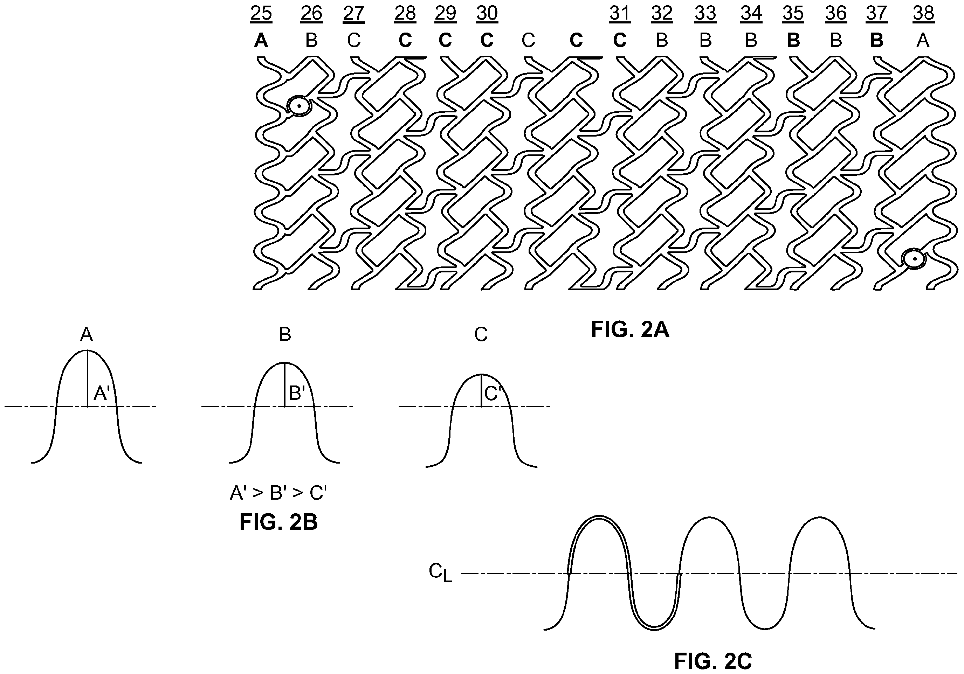

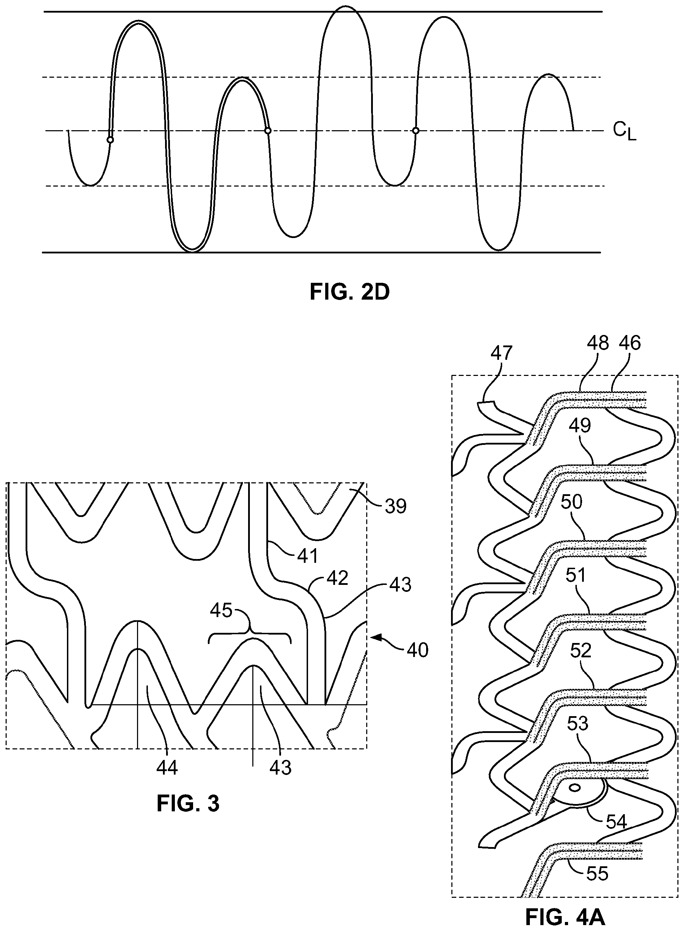

A second embodiment of the scaffold is shown in Figure 2A, here, the circumferential elements of the scaffold vary in length. The circumferential length of the circumferential elements are A>B>C; however, this order of lengths is shown only for illustration purposes. The amplitude of the undulations formed by the circumferential elements A, B, C, in a partially crimped or fully crimped state also has the length or height of A'>B'>C' (Figure 2B). In the embodiment shown, the circumferential elements with varying lengths are distributed along the body of the scaffold as follows: A-B (25, 26), C-C (27, 28), C-C (29, 30), C-B (31, 32), B-B (33, 34), B-B (35, 36), and B-A (37, 38). However, numerous other combinations and distributions are possible as well. Figure 2C shows a repeating sinusoidal pattern where the amplitude of the undulations is constant across the circumferential element. Figure 2D shows a non-repeating sinusoid pattern. In a non-repeating sinusoid pattern, the amplitude and/or ordinary frequency of the undulations may vary either systematically or randomly along the circumference of the circumferential element.

The length of a circumferential element refers to the absolute distance of travel along the circumferential element starting from an artificial point on the circumferential element and back around to the same artificial point.

A detailed view of one part of the undulations forming two adjacent pairs of circumferential elements is shown in Figure 3. The circumferential elements are shown as 39, 40. They are connected by a connection element (second connection element), which has two linear portions, 41, 43 and an S-shaped portion 42. The undulations of the circumferential element 40 have amplitudes 43 and 44. In the embodiment shown, 44 is greater than 43.

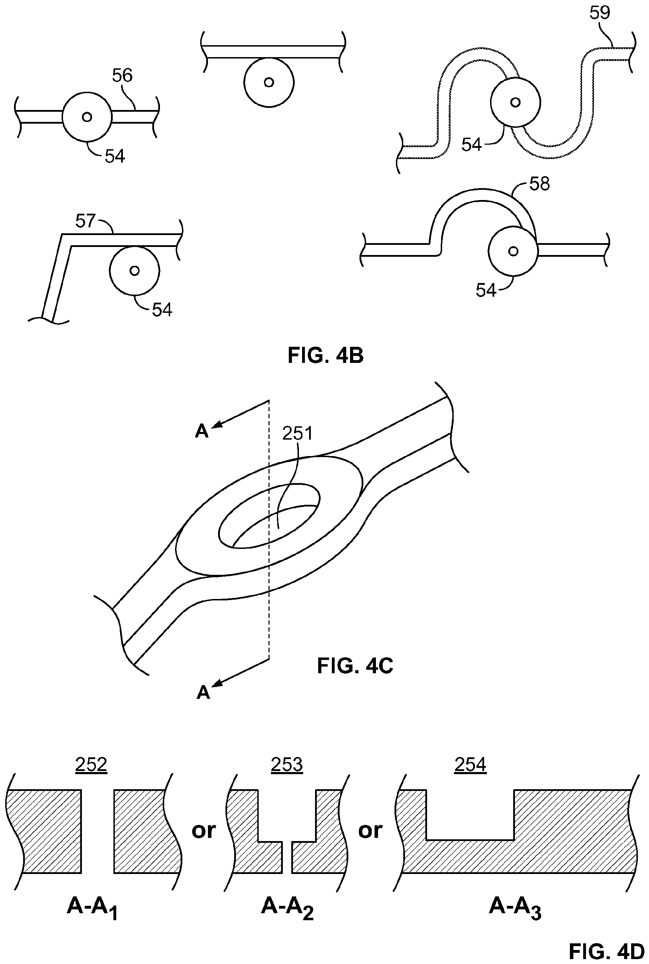

Figure 4A shows the details of the connection elements (first connection elements) at one end of the scaffold. Circumferential elements 46, 47 are connected by connection elements (first connection elements) 48-55. Figure 4B illustrates various embodiments of where the marker dot, 54, can be attached to the connection element, 56-59, at various points along the connection element 53. Figure 4C shows a perspective view of a marker dot 251. Figure 4D shows cross sections of various embodiments of marker dot 252. The section of a scaffold for a marker dot can take various forms, including, but not limited to, a see-through void (252), a cup-shaped structure with a hole in the bottom (253), and a cup-shaped structure (254).

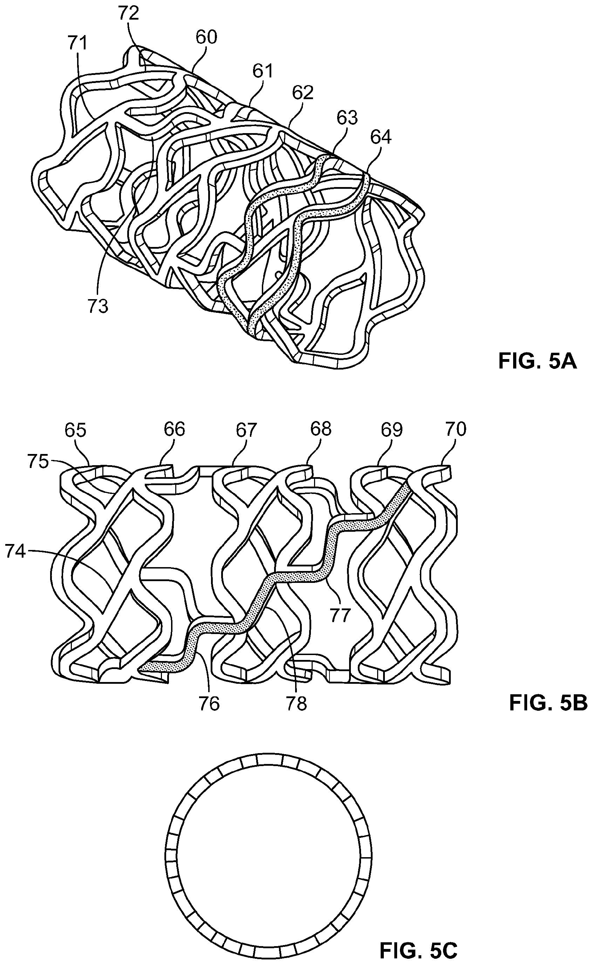

Figures 5A-5C show the scaffold in an orthogonal (5A), side (5B) and end (5C) views. The circumferential elements are labeled 60-64 and 65-70. The connection elements between pairs of circumferential elements are shown as 71, 72, 74, 75 (first connection element), while the connection element between two pairs is labeled 73. The formation of the spiral design is shown as 76-78 and contains the connection elements between pairs of circumferential elements as well as the connection elements between circumferential elements forming the pair (first and second connection elements, respectively). The circumferential elements forming a pair are shown with highlighted lines 63, 64.

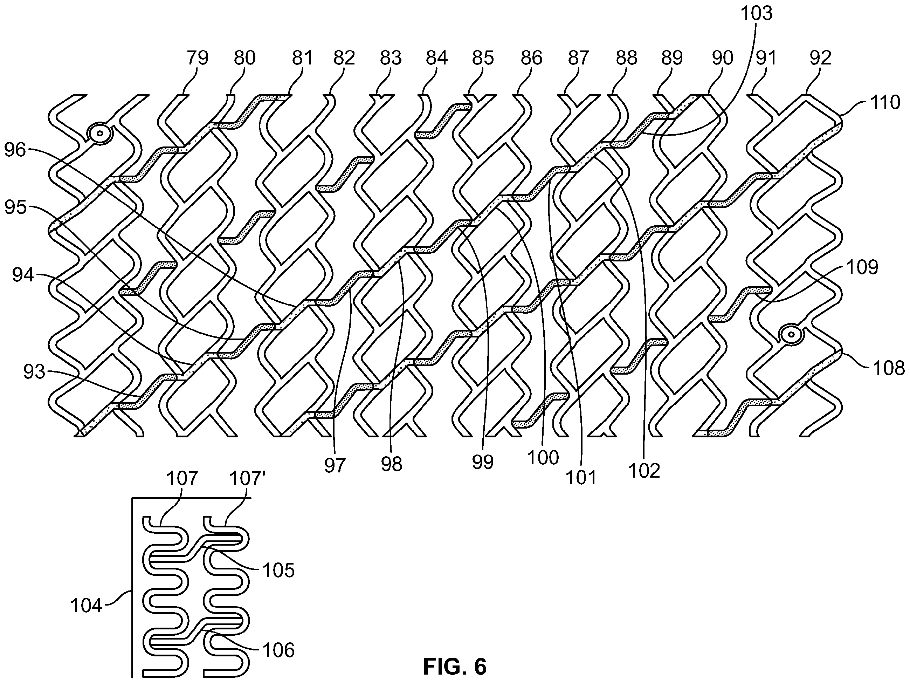

Figure 6 shows a cut pattern of another embodiment of the scaffold. The circumferential elements are labeled 79-92, with the pairs of circumferential elements being shown as 79,80; 81,82; 83,84; 85,86; 87, 88; 89,90 and 91, 92. The spiral pattern is shown as 93-103 and comprises an alternating pattern of connection elements between pairs of circumferential elements (second connection elements), 93, 95, 97, 99, 101 and 103, with connection elements between the circumferential elements forming a pair (first connection elements), 94, 96, 98, 100 and 102. In the inset to Figure 6, a partially compressed view of the scaffold is shown. The circumferential elements are labeled 107, 107', while the connection elements are labeled 105, 106. The scaffold shown in the figure contains more than one spiral pattern, 108-110 which may be substantially parallel to one another.

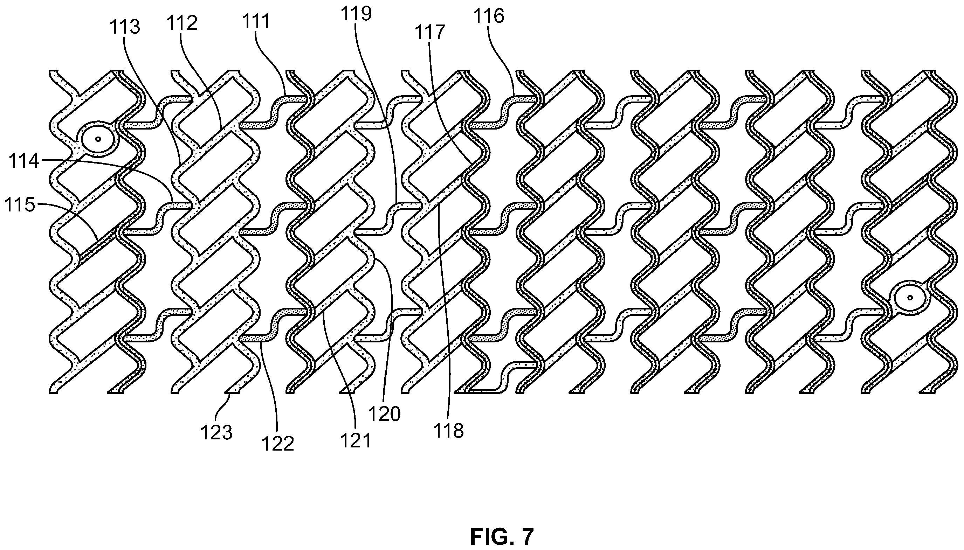

Figure 7 shows a cut pattern of the scaffold with an alternative spiral pattern. In this embodiment, the spiral pattern is formed from an alternating pattern of connection elements between pairs of circumferential elements (first connection elements), 112, 115, 118, 121, portions of the undulations of the circumferential elements, 113, 117, 120, and connection elements connecting two adjacent pairs of circumferential elements (second connection elements), 111, 114, 116, 119, 122. In this embodiment, the spiral pattern has the following repeating sequence starting from one end of the scaffold: first connection element, second connection element, portion of the undulation, first connection element, second connection element, portion of the undulation, which is repeated across the scaffold. Other repeating sequences are possible, including, but not limited to, (a) first connection element, portion of the undulation, first connection element, repeated n times across the body of the scaffold; (b) first connection element, portion of the undulation, repeated n times across the body of the scaffold; (c) second connection element, portion of the undulation, repeated n times across the body of the scaffold; or (d) second connection element, portion of the undulation, second connection element, repeated n times across the body of the scaffold;

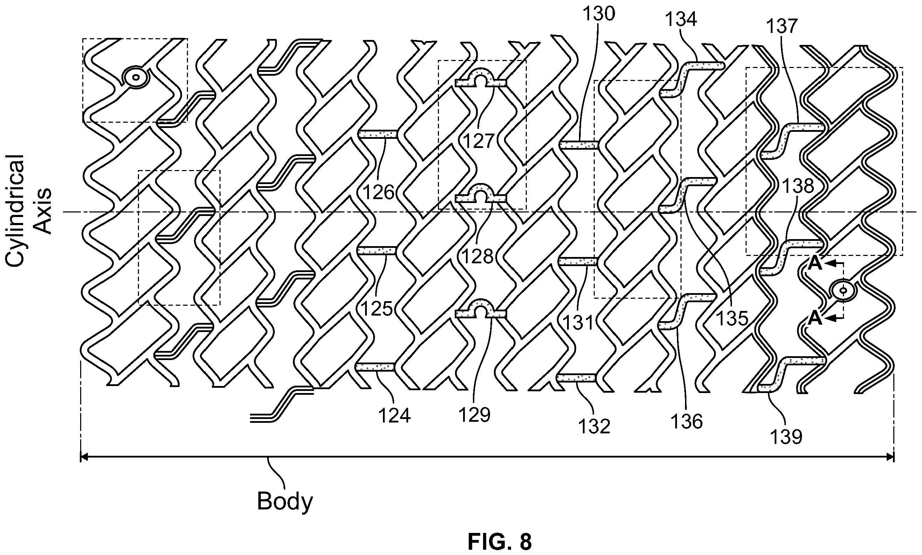

Figure 8 shows an embodiment where the connection elements have a variety of different shapes and the distribution of the pattern of the connection elements varies across the body of the scaffold. Specifically, in the embodiment shown, the connection elements between pairs of circumferential elements, are linear, 124-126 and 130-132 or curvilinear, 127-129, 134-136 and 137-139. One of the curvilinear connection elements between pairs of circumferential elements have both linear and curvilinear portions, 127-129. As is evident from the illustration, the pattern of connection elements can vary across the body of the scaffold, curvilinear, 137-139, 134-136, linear, 130-132, curvilinear, 127-129 and linear, 124-126. Other possible arrangements as well as geometric shapes are encompassed by the invention. One of ordinary skill in the art can select the sequence and type of connection elements to fit the flexibility and spatial requirements posed by the vasculature.

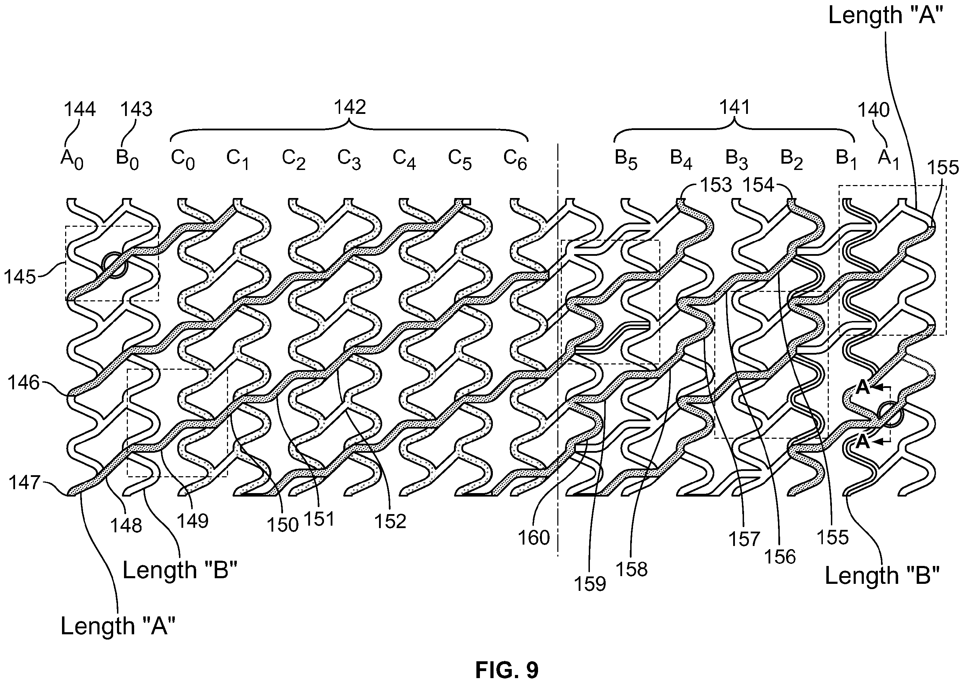

Figure 9 shows a cut pattern of the scaffold with an alternative spiral pattern together with circumferential elements of varying length. This embodiment illustrates the modular design nature of the scaffold where circumferential elements of differing lengths as well as a variety of different connection elements are combined to form a scaffold having two different spiral patterns. The circumferential elements have lengths, Aₙ, Bₙ and Cₙ. For example, in one embodiment, the circumferential lengths are A>B>C. Specifically, A1 and A0 (140, 144) are combined with B₀-B₅ (141, 143) which in turn are combined with C₀-C₆ (142) in the sequence shown in Figure 9. There are two different spiral patterns present in this embodiment, 145-147, and 153-154. The spiral pattern in 147 comprises a connection element between the circumferential elements forming the pair (first connection elements), 148, 150, 152 and the connection element between pairs of circumferential elements (second connection elements), 149, 151. The other spiral pattern, 154, comprises a connection element between the circumferential elements forming the pair (first connection elements), 155, 158, a portion of the undulation of the circumferential element, 157, 160 and the connection element between pairs of circumferential elements (second connection elements), 156, 159.

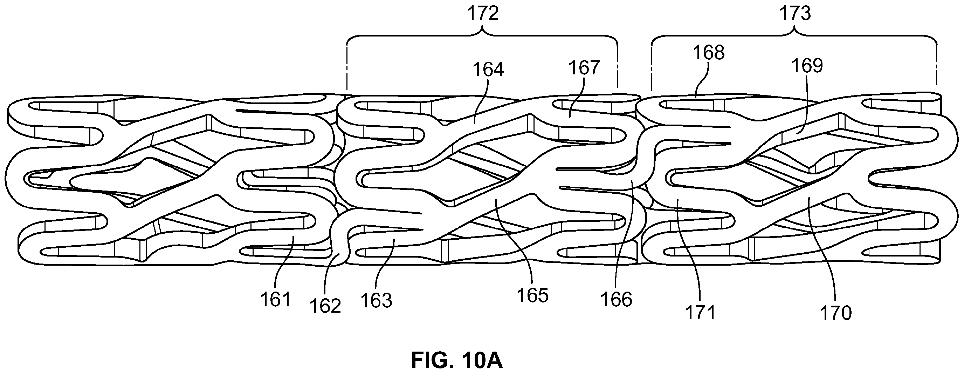

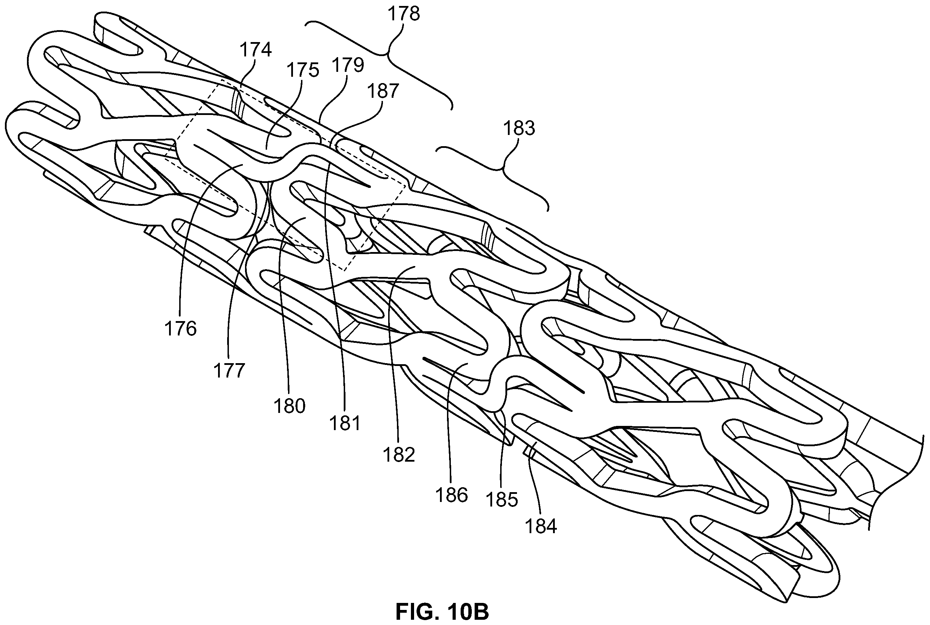

Figures 10A and 10B show side views of a three-dimensional perspective of the scaffold in a crimped or compressed state. The pairs of circumferential elements are shown as 172,173. One undulation forming part of a circumferential element 161,167 is adjacent to a connection element between the pairs of circumferential elements 162, 166 (the second connection element). This connection element sits on top or nestles/nests, but does not necessarily touch, the undulation of a circumferential element in an adjacent pair 163, 171. The connection elements connecting the circumferential elements in a pair are shown as 169, 170 (the first connection element). This arrangement can also be seen clearly in Figure 10B where one undulation forming part of a circumferential element 175,186 is adjacent to a connection element between the pairs of circumferential elements 176, 185 (the second connection element). This connection element sits on top or nestles/nests the undulation of a circumferential element in an adjacent pair 180, 184. The connection elements connecting the circumferential elements in a pair are shown as 182 (the first connection element). A pair of circumferential elements is labeled as 183.

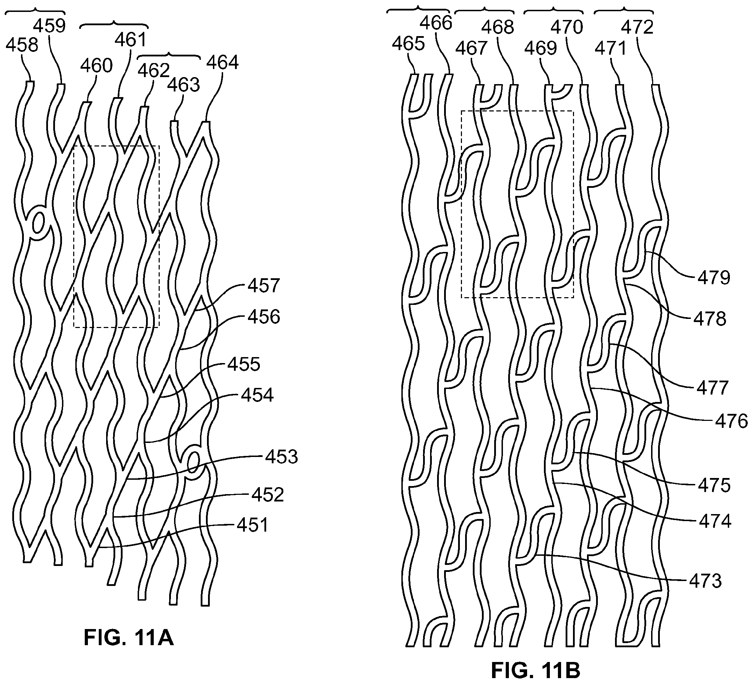

Figure 11A shows a cut pattern of the scaffold with an alternative spiral pattern. The circumferential elements are labeled 458-464, with the pairs of circumferential elements being shown as 458, 459; 460, 461 and 462, 463. In this embodiment, the spiral pattern is formed from an alternating pattern of connection elements between pairs of circumferential elements (first connection elements), 451, 455, portions of the undulations of the circumferential elements, 452, 454, 456, and connection elements connecting two adjacent pairs of circumferential elements (second connection elements), 453, 457. In this embodiment, the spiral pattern has the following repeating sequence starting from one end of the scaffold: first connection element, portion of the undulation, second connection element, portion of the undulation, which is repeated across the scaffold. In Figure 11A, both the first and second connection elements are linear.

Figure 11B shows a cut pattern of the scaffold with another spiral pattern. The circumferential elements are labeled 465-472, with the pairs of circumferential elements being shown as 465, 466; 467, 468; 469, 470 and 471, 472. In this embodiment, the spiral pattern is formed from an alternating pattern of connection elements connecting two adjacent pairs of circumferential elements (second connection elements), 473, 477, portions of the undulations of the circumferential elements, 474, 476, 478, and connection elements between pairs of circumferential elements (first connection elements), 475, 479. In this embodiment, the spiral pattern has the following repeating sequence starting from one end of the scaffold: second connection element, portion of the undulation, first connection element, portion of the undulation, which is repeated across the scaffold. In Figure 11B, both the first and second connection elements are S-shaped.

One of the issues with the prior art designs is that, when the scaffold expands, the undulations forming adjacent circumferential elements are distorted. The present design is an improvement.

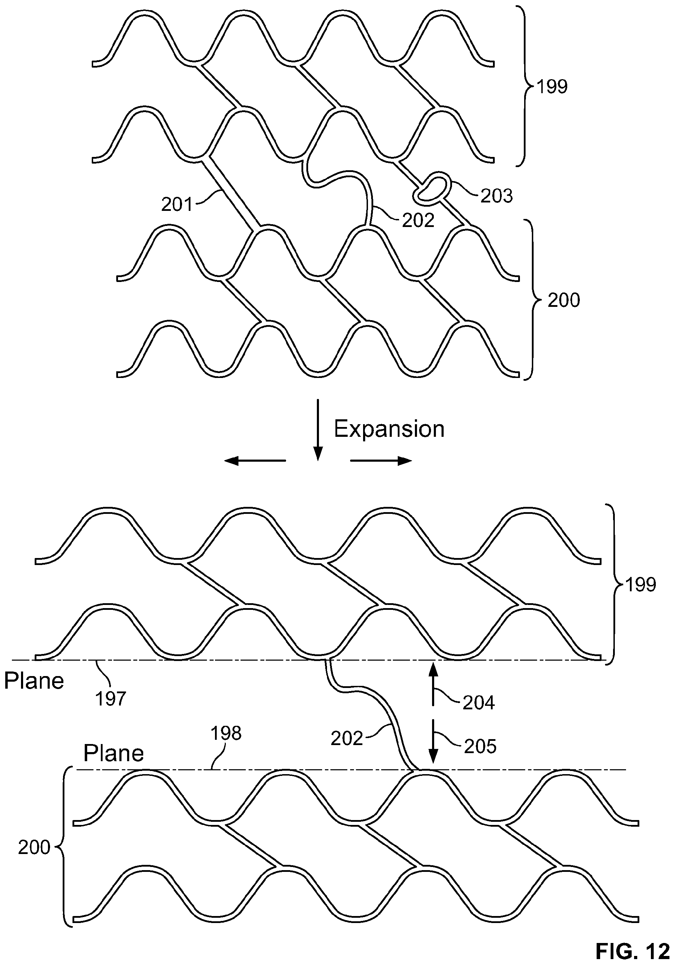

Figure 12 illustrates the functionality of the connection element between adjacent pairs of circumferential elements. 199 and 200 are pairs of circumferential elements. Three types of connection elements are illustrated, linear, 201 and curvilinear 202, 203. When the scaffold expands, connection element 201 is restrictive and cannot accommodate expansion of the circumferential elements; however, either connection element 203 or 203 are less restrictive and accommodate expansion without distorting the circumferential elements in adjacent pairs out of the plane, 197, 198.

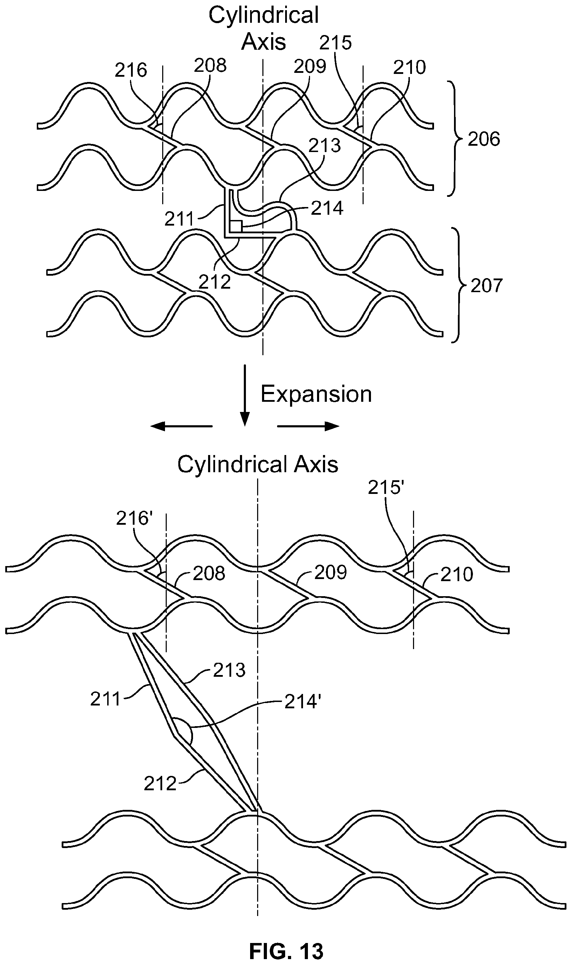

Figure 13 illustrates the geometry of the scaffold and the connection elements on expansion. The pairs of circumferential elements are shown as 206, 207. The connection elements between the undulations (first connection element) forming the pairs of circumferential elements are labeled 208-210. The connection element between pairs is labeled 213. The geometry of expansion is illustrated by creating a triangle with 213 using 211,212. The angle formed with respect to the cylindrical axis for 208 and 210 is labeled 215, 216. The angle formed with 211, 212 and 213 is labeled 214. After expansion, the angles 214', 215' and 216' all increase.

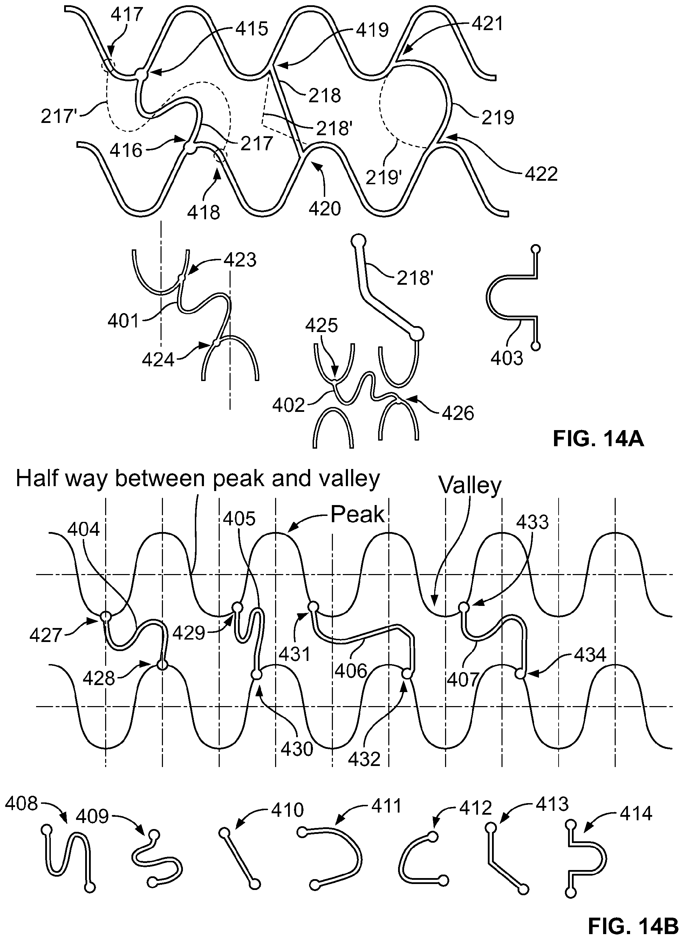

Figures 14A and 14B illustrate various embodiments of the structural geometry of connection elements which may be between circumferential elements in a pair (first connection elements) or between adjacent pairs of circumferential elements (second connection elements). The connection elements can be S-shaped, 217, 217', 401, 404 - 409, linear, 218, 410, 218', 413 (bent), concave/convex 219, 219', 411, 412, curvilinear, 402, 403, 414. The connection elements may connect any points on adjacent circumferential elements, including, peak (426, 428,), valley (425, 427,), or any point on the ascending portion (415, 416, 419, 420, 421, 422, 423, 424, 429, 430, 433) or descending portion (417, 418, 431, 432, 434) of an undulation.

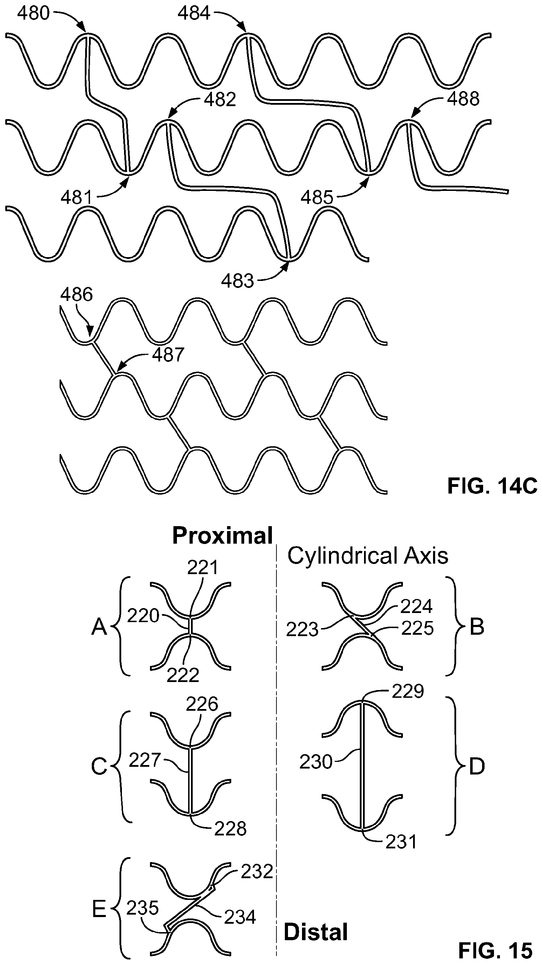

Figure 14C shows that, with one end of the connection elements being attached to a point on an undulation of a circumferential element, the other end of the connection element may be attached to the direct corresponding peak or valley of the adjacent circumferential element, or to a point shifted by 1, 2, 3, 4, 5, 6, 7, 8, 9 ... N (N can be any positive integers) undulations (the shift may be towards either directions). In Figure 14C, the two ends of the connection elements are: 480 (peak), 481 (valley); 482 (peak), 483 (valley shifted by 1 undulation); 484 (peak), 485 (valley shifted by 1 undulation); 486 (valley), 487 (peak).

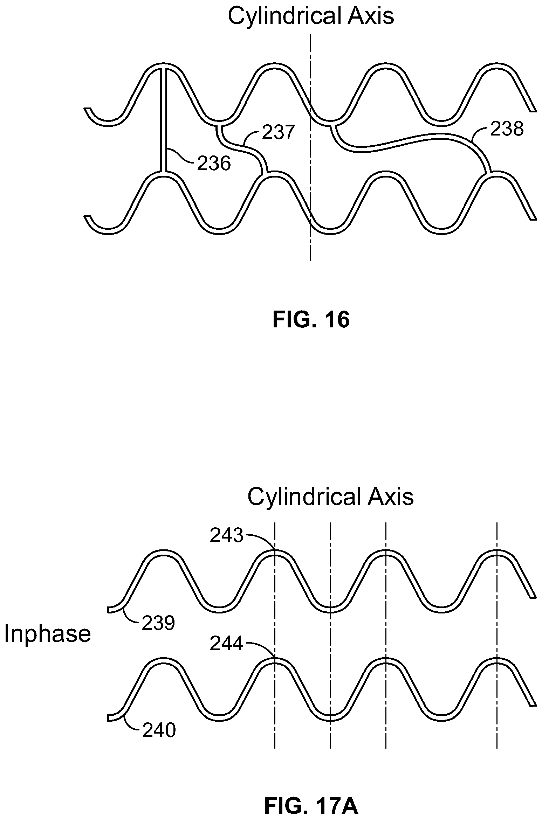

Figures 15A-E illustrates where the connection elements can attach along the undulations. The connection elements here are shown as linear; however, this shape is only shown for illustration purposes and the connection elements can be curvilinear or S-shaped or any other shape encompassed by the invention. The embodiments shown here can apply to both the first and second connection elements. The cylindrical axis of the scaffold is shown as well. The connection element 220 can be attached to the valley of one undulation, 221, and the peak of another undulation, 222 (Figure 15A). Alternatively, the connection elements 224 can be attached on either side of the valley or peak of two undulations 223, 225 (Figure 15B). The connection element 227 can be attached to the valleys 226, 228 of two undulations (Figure 15C). The connection element 230 can also be attached to the peak 229, and valley 231 of two undulations (Figure 15D). The connection element 234 can also be attached to the ascending portions 232, 235 of two undulations (Figure 15E). The figures shown here illustrate the connection element attached to an opposing undulation; however, the connection element can be attached to undulations that are shifted radially with respect to the cylindrical axis (Figure 16) (236-238).

The proximal and distal ends of the scaffold are labeled with respect to the heart with the proximal end closest to the aortic valve. The terms peak and valley are arbitrarily defined with respect to the proximal and distal ends of the scaffold.

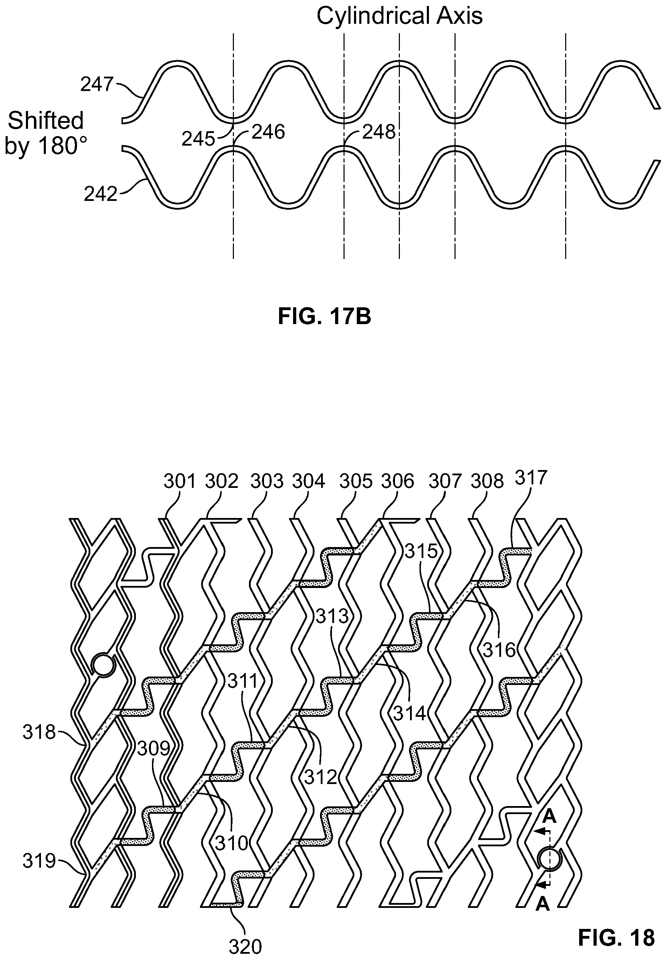

Figures 17A and 17B show an example of phasing in adjacent circumferential elements. In Figure 17A, the circumferential elements 239, 240 are in-phase with each other (compare cylindrical axial lines for 243 and 244). In contrast, in Figure 17B, the circumferential elements 242, 247 are 180° out-of-phase with each other (compare 245 and 248).

Figure 18 shows a cut pattern of another embodiment of the scaffold. The circumferential elements are labeled 301-308, with the pairs of circumferential elements being shown as 301, 302; 303, 304; 305, 306; and 307, 308. The spiral pattern is shown as 309-317 and comprises an alternating pattern of connection elements between pairs of circumferential elements (second connection elements), 309, 311, 313, 315 and 317, with connection elements between the circumferential elements forming a pair (first connection elements), 310, 312, 314 and 316. The scaffold shown in the figure contains more than one spiral pattern, 318-320.

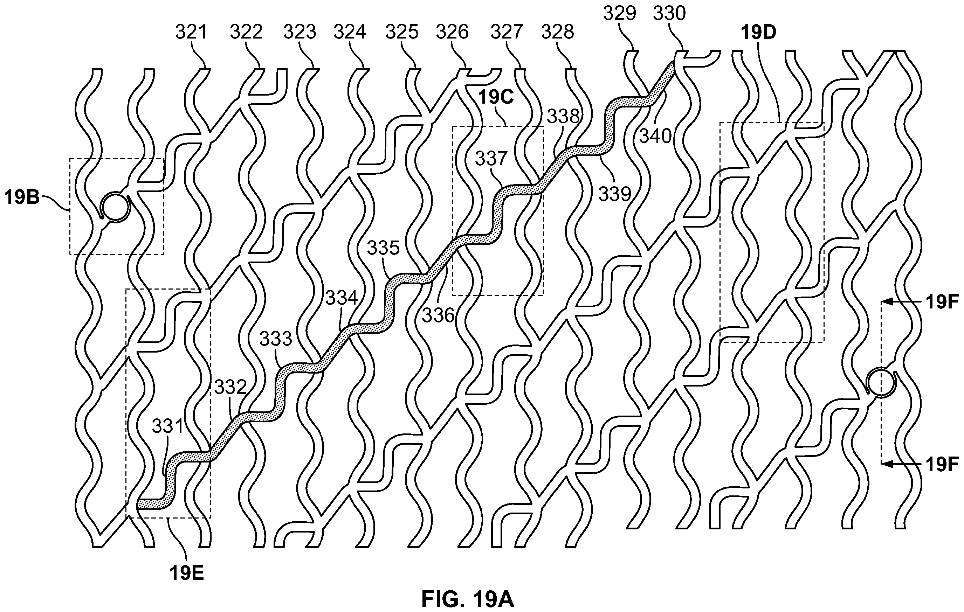

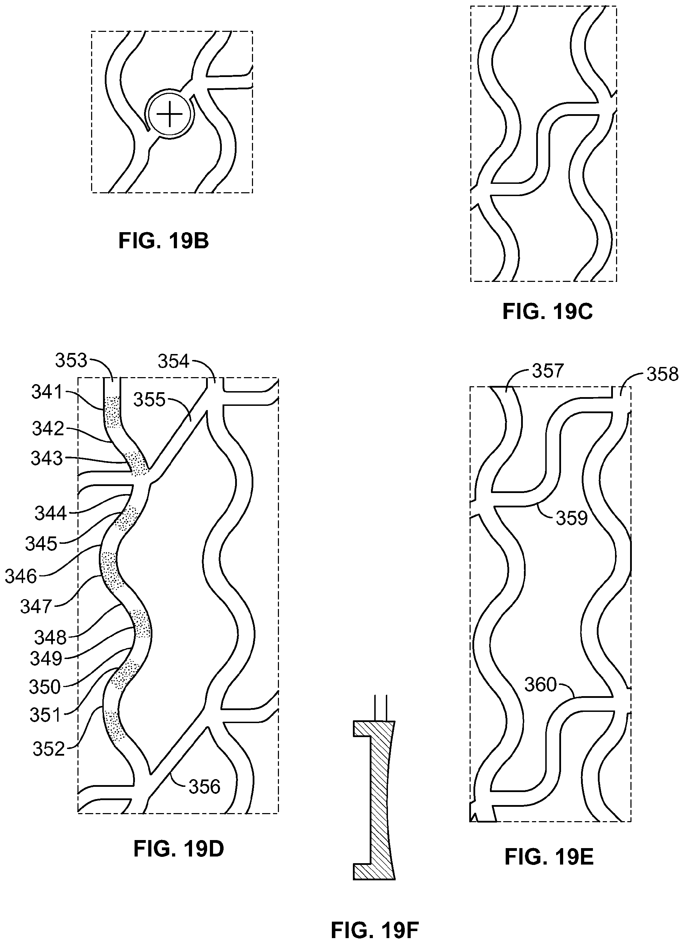

Figure 19A shows a cut pattern of another embodiment of the scaffold. The circumferential elements of this embodiment are comprised of a plurality of linear segments. The circumferential elements are labeled 321-330, with the pairs of circumferential elements being shown as 321, 322; 323, 324; 325, 326; 327, 328 and 329, 330. The spiral pattern is shown as 331-340 and comprises an alternating pattern of connection elements between pairs of circumferential elements (second connection elements), 331, 333, 335, 337 and 339, with connection elements between the circumferential elements forming a pair (first connection elements), 332, 334, 336, 338 and 340. In the spiral or helical patterns shown the spiral pattern cuts-across the circumferential elements.

Detailed views of parts of the scaffold in Figure 19A are shown in Figures 19B - 19E. In Figure 19D, the circumferential elements in a pair are shown as 353, 354. They are connected by connection elements (first connection elements), 355, 356. In Figure 19D, a circumferential element is shown to be comprised of a plurality of linear segments, 341 - 352. In Figure 19E, circumferential elements 357, 358 are connected by connection elements (second connection elements) 359, 360.

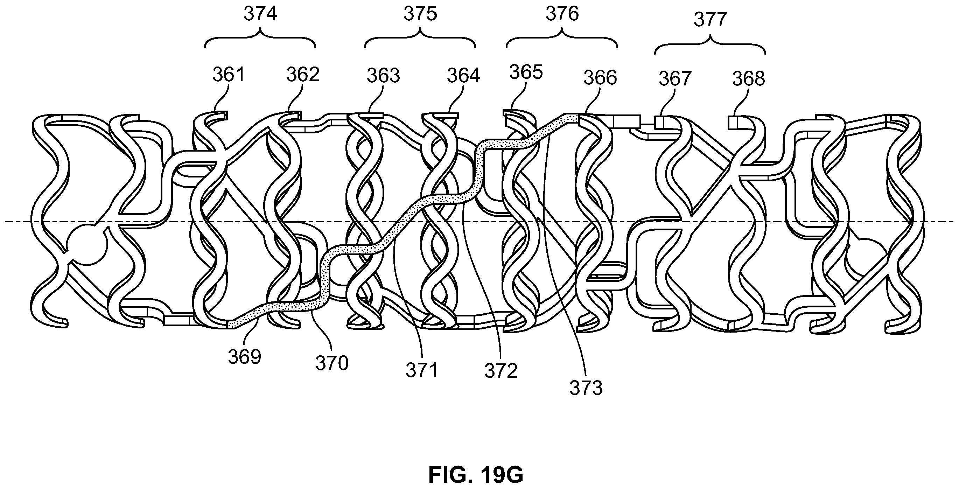

Figure 19G shows a side view of the scaffold. The circumferential elements are labeled 361-368, forming pairs 374-377. The connection elements between circumferential elements within a pair (first connection element) are shown as 369, 371, 373, while the connection element between two pairs of circumferential elements are labeled 370, 372. The formation of the spiral design is shown as 369-373 and contains the connection elements between adjacent pairs of circumferential elements (second connection elements) as well as the connection elements between circumferential elements forming the pair (first connection elements).

Scaffolds of the present invention may employ one, two or more end zones. The end zone may take numerous forms. An end zone may be formed from one or a plurality of circumferential elements and is connected to the main body at one or more bridging elements. Adjacent circumferential elements may be connected directly, or may be connected by at least one second connection element. In one embodiment, an end zone contains a first and second circumferential element.

The scaffold may further comprise at least one radiopaque marker. See, www.nitinol-curope.com/pdfs/stentdesign.pdf for a review of the design and makeup of radiopaque markers which are well known in the art. The radiopaque markers may assume a variety of different sizes and shapes. For example, a radiopaque marker may contain a centrally placed marker hole. The marker hole may be circular or semicircular, but may also assume other shapes, such as a semicircular hole with an extrusion or dimple positioned at one portion of the circumference, or a hole in the shape of a heart.

The radiopaque marker may be electron-dense or x-ray refractile markers, such as metal particles or salts. Non-limiting examples of suitable marker metals include iron, gold, colloidal silver, zinc and magnesium, either in pure form or as organic compounds. Other radiopaque materials are tantalum, tungsten, platinum/iridium, or platinum. Heavy metal and heavy earth elements are useful in variety of compounds such as ferrous salts, organic iodine substances, bismuth or barium salts, etc. Further embodiments that may be utilized may encompass natural encapsulated iron particles such as ferritin that may be further cross-linked by cross-linking agents. Ferritin gel can be constituted by cross-linking with low concentrations (0.1-2%) of glutaraldehyde. The radiopaque marker may be constituted with a binding agent of one or more biodegradable polymer, such as PLLA, PDLA, PLGA, PEG, etc. In one embodiment comprising a radiopaque marker, iron containing compounds or iron particles are encapsulated in a PLLA polymer matrix to produce a pasty substance, which can be injected or otherwise deposited in the hollow receptacle contained about the stent.

The scaffolds may also have a transition zone between the end zone and the main body. The transition zone may be formed from a plurality of undulations, each undulation comprising two adjacent connection elements connected by a loop with the width of the loop varying across the transition zone. The transition zone may comprise a plurality of polygons where the surface area of adjacent polygons in the transition zone increases circumferentially. U.S. Patent Publication No. 20110125251. The transition zone may take other suitable configurations.

The dimensions of the scaffold may vary from about 10 mm to about 300mm in length, from 20 mm to about 300 mm in length, from about 40 mm to about 300 mm in length, from about 20 mm to about 200 mm in length, from about 60 mm to about 150 mm in length, or from about 80 mm to about 120 mm in length. The internal diameter (I.D.) of the stent may range from about 2 mm to about 25 mm, from about 2 mm to about 5 mm (e.g., for the coronary arteries), from about 4 mm to about 8 mm (e.g., for neurological spaces in the CNS, both vascular and nonvascular), from about 6 mm to about 12 mm (e.g., for the iliofemoral), from about 10mm to about 20 mm (e.g., for the ilioaortic) and from about 10 mm to about 25 mm (e.g., for the aortic).

The device of the present invention may be used as a self-expanding stent or with any balloon catheter stent delivery system, including balloon catheter stent delivery systems described in U.S. Patent Nos. 6,168,617, 6,222,097, 6,331,186 and 6,478,814. In one embodiment, the present device is used with the balloon catheter system disclosed in U.S. Patent No. 7,169,162.

The scaffold of the present invention may be used with any suitable catheter, the diameter of which may range from about 0.8mm to about 5.5 mm, from about 1.0 mm to about 4.5 mm, from about 1.2 mm to about 2.2 mm, or from about 1.8 to about 3 mm. In one embodiment, the catheter is about 6 French (2 mm) in diameter. In another embodiment, the catheter is about 5 French (1.7 mm) diameter.

The scaffold may be inserted into the lumen of any vessel or body cavity expanding its cross-sectional lumen. The invention may be deployed in any artery, vein, duct or other vessel such as a ureter or urethra and may be used to treat narrowing or stenosis of any artery, including, the coronary, infrainguinal, aortoiliac, subclavian, mesenteric or renal arteries. Other types of vessel obstructions, such as those resulting from a dissecting aneurysm are also encompassed by the invention. The subjects that can be treated using the scaffolds and methods of this invention are mammals, including a human, horse, dog, cat, pig, rabbit, rodent, monkey and the like.

The scaffold of the present invention may be formed from at least one bioabsorbable polymer representing a wide range of different polymers which is capable of crystallizing. Typically, bioabsorbable polymers comprise aliphatic polyesters based on lactide backbone such as poly L-lactide (PLLA), poly D-lactide (PDLA), poly D,L-lactide, mesolactide, glycolides, lactones, as homopolymers or copolymers, as well as formed in copolymer moieties with comonomers such as, trimethylene carbonate (TMC) or ε-caprolactone (ECL). U.S. Patent Nos. 6,706,854 and 6,607,548; EP 0401844; and Jeon et al. Synthesis and Characterization of Poly (L-lactide) - Poly (ε-caprolactone). Multiblock Copolymers Macromolecules 2003: 36, 5585 - 5592. The copolymers comprises a moiety such as L-lactide or D-lactide of sufficient length that the copolymer can crystallize and not be sterically hindered by the presence of glycolide, polyethylene glycol (PEG), ε-caprolactone, trimethylene carbonate or monomethoxy-terminated PEG (PEG-MME). For example, in certain embodiments greater than 10, 100 or 250 L or D-lactides may be arrayed sequentially in the polymer. The stent may also be composed of bioabsorbable polymeric compositions such as those disclosed in U.S. Patent Nos. 7,846,361; 7,897,224 and 8,137,603; and applicant's co-pending U.S. Patent Publication No. 2010/0093946.

Based on the presence of the monomer type, the following polymer nomenclature can be used.

| | |
|---|---|
| LPLA: | Poly(L-lactide) |
| LPLA-PEG: | Poly(poly-L-lactide-polyethylene glycol) |
| DPLA: | Poly(D-lactide) |
| DPLA-TMC: | Poly(poly D-lactide-co-trimethylene carbonate) |
| DLPLA: | Poly(DL-lactide), a racemic copolymer D-co-L-lactide |
| LDPLA: | Poly(L-co-D-lactide) |
| LDLPLA: | Poly(L-co-DL-lactide), named for the method of monomer introduction |
| PGA: | Poly(glycolide) |
| PDO: | Poly(dioxanone) (PDS is Trademark) |
| SR: | "Self reinforced" (a processing technique) |
| TMC: | Trimethylene carbonate |
| PCL: | Poly(e-caprolactone) |
| LPLA-TMC: | Poly(poly L-lactide-co-trimethylene carbonate) |
| LPLG: | Poly(L-lactide-co-glycolide) |
| POE: | Poly Orthoester |

In an embodiment of the present invention, the composition comprises a base polymer of poly(L-lactide) or poly(D-lactide). Other base polymer compositions include blends of poly(L-lactide) and poly(D-lactide). Other advantageous base polymer compositions include poly(L-lactide-co-D,L-lactide) or poly(D-lactide-co-D,L-lactide) with a D,L-lactide co-monomer molar ratio from 10 to 30%, and poly(L-lactide-co-glycolide) or poly(D-lactide-co-glycolide) with a glycolide co-monomer molar ratio from 10 to 20%.

Another embodiment embodies a base polymer featuring a poly (L-lactide) moiety, and/or a poly (D-lactide) moiety, linked with a modifying copolymer thereof, including poly (L-lactide-co-tri-methylene-carbonate or poly(D-lactide-co-tri-methylene-carbonate) and (L-lactide-co-ε-caprolactone), or poly(D-lactide-co-ε-caprolactone), in the form of block copolymers or blocky random copolymers, wherein the lactide chain length is sufficient to effect cross-moiety crystallization. Cross moiety crystallization of compositions with copolymers affords increased modulus data in tensile tests avoiding the method for reducing the tensile strength in the polymer blend.

The polymer composition can allow for the development of the lactide racemate (stereo complex) crystal structure, between the L and D moieties, to further enhance the mechanical properties of the bioabsorbable polymer medical device. The formation of the racemate (stereo complex) crystal structure can accrue from formulations such as combinations of:
Poly L-lactide with Poly D-lactide with Poly L-lactide-co-TMC;
Poly D-lactide with Poly L-lactide-co-TMC;
Poly L-lactide with Poly D-lactide-co-TMC;
Poly L-lactide with Poly D-lactide with Poly D-lactide-co-TMC;
Poly L-lactide-co-PEG with Poly D-lactide-co-TMC; and
Poly D-lactide-co-PEG with Poly L-lactide-co-TMC.

Poly-lactide racemate compositions can be "cold formable or bendable" without adding additional heat. Cold-bendable scaffolds of the invention do not require beating to become flexible enough to be crimped onto a carrier device or accommodate an irregularly shaped organ spaces. Cold bendable ambient temperatures are defined as room temperature not exceeding 30°C. Cold-bendable scaffolds can afford sufficient flexibility when implanted allowing for an expanded scaffold device in an organ space such as pulsating vascular lumen. For example, in terms of a stent, it may be desirable to utilize polymeric compositions that afford mostly amorphous polymer moieties after fabrication that can crystallize particularly when the secondary nested or end-positioned meandering connection elements when the scaffold is strained by stretching upon balloon expansion for implantation. Such cold-bendable polymeric scaffold embodiments of are not brittle and do not have to be preheated to a flexible state prior to implantation onto a contoured surface space in the body. Cold-bendability allows these blends to be crimped at room temperature without crazing, and moreover, the blends can be expanded at physiological conditions without crazing.

Poly-lactide racemate compositions and non-racemate compositions of embodiments herein may be processed to have blocky moieties allowing cross moiety crystallization even with the addition of an impact modifier to the blend composition. Such a blend introduces the possibility to design device specific polymer compositions or blends by producing either single or double Tg's (glass melt transition points).

As is understood in this art, polymer compositions of the present invention can be customized to accommodate various requirements of the selected medical device. The requirements include mechanical strength, elasticity, flexibility, resilience, and rate of degradation under physiological and localized anatomical conditions. Additional effects of a specific composition concern solubility of metabolites, hydrophilicity and uptake of water and any release rates of matrix attached or enclosed pharmaceuticals.

The polymer implant utility can be evaluated by measuring mass loss, decrease in molecular weight, retention of mechanical properties, and/or tissue reaction. More critical for scaffold performance are hydrolytic stability, thermal transitions crystallinity and orientation. Other determinants negatively affecting scaffold performance include, but not exclusively, monomeric impurities, cyclic and acyclic oligomers, structural defects and aging.

The scaffold fashioned from the polymer compositions above may be significantly amorphous post extrusion or molding. The scaffold may be subjected to controlled re-crystallization to induce incremental amounts of crystallinity and mechanical strength enhancement. Further crystallization can be induced by strain introduction at the time of device deployment. Such incremental re-crystallization may be employed either on a scaffold "blank" prior to secondary or final fabrication (such as by laser cutting) or post such secondary fabrication. Crystallization (and thus mechanical properties) can also be maximized by strain induction such as by "cold" drawing polymeric tubing, hollow fiber, sheet or film, or monofilament prior to further fabrication. Crystallinity has been observed to contribute a greater stiffness in the scaffold. Therefore, the polymer composition and steric complex of the scaffold has both amorphous and paracrystalline moieties. The initially semicrystalline polymer portion can be manipulated by the action of stretching or expansion of a given device. Yet an adequate amount of amorphous polymeric character is desirable for flexibility and elasticity of the polymeric device. The usual monomer components include lactide, glycolide, caprolactone, dioxanone, and trimethylene carbonate. The scaffold may also be constructed to allow relatively uniform exposure to local tissue or circulatory bioactive factors and enzymes perfusing and acting on the polymer structure during bioabsorption.

The rate of in situ breakdown kinetics of the polymeric matrix of an organ space implant, such as a cardiovascular stent, is sufficiently gradual to avoid tissue overload, inflammatory reactions or other more adverse consequences. In an embodiment, the scaffold is fabricated to survive at least 1, 2, 3, 4, 5, 6, 7, 8, 9, 10, 11, 12, 24 or 36 months.

Pharmaceutical compositions may be incorporated within the polymers by, for example, grafting to the polymer active sites, or coating. An embodiment of the polymer according to the invention affords attachment or incorporation the biological healing factors or other drugs in the polymeric matrix or a polymer coating.

In another embodiment, the composition may be constructed to structurally enclose or attach to drugs in the polymeric matrix. The purpose of such additives may to provide, for example with respect to a stent, treatment of the cardiovascular system or in vascular site in contact with the medical device polymer. The kind of enclosure or attachment of drugs in the polymer may determine the rate of release form the scaffold. For example, the drug or other additive may be bound in the polymer matrix by various known methods including but not limited to covalent bonds, non-polar bonds as well as an ester or similar bioreversible bonding means.

In one embodiment, a bioabsorbable implantable medical device may be covered with a biodegradable and bioabsorbable coating containing one or more barrier layers where the polymer matrix contains one or more of the aforementioned pharmaceutical substances. In this embodiment, the barrier layer may comprise a suitable biodegradable material, including but not limited to, suitable biodegradable polymers including: polyesters such as PLA, PGA, PLGA, PPF, PCL, PCC, TMC and any copolymer of these; polycarboxylic acid, polyanhydrides including maleic anhydride polymers; polyorthoesters; poly-amino acids; polyethylene oxide; polyphosphacenes; polylactic acid, polyglycolic acid and copolymers and mixtures thereof such as poly(L-lactic acid) (PLLA), poly(D,L-lactide), poly(lactic acid-co-glycolic acid), 50/50 (DL-lactide-co-glycolide); polydixanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethyl-carbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate. The number of barrier layers that the polymeric scaffold on a device may have depends on the amount of therapeutic need as dictated by the therapy required by the patient. For example, the longer the treatment, the more therapeutic substance required over a period of time, the more barrier layers to provide the pharmaceutical substance in a timely manner.

In another embodiment, the additive in the polymer composition may be in the form of a multiple component pharmaceutical composition within the matrix such as containing a last release pharmaceutical agent to retard early neointimal hyperplasia/smooth muscle cell migration and proliferation, and a secondary biostable matrix that releases a long acting agent for maintaining vessel patency or a positive blood vessel remodeling agent, such as endothelial nitric oxide synthase (eNOS), nitric oxide donors and derivatives such as aspirin or derivatives thereof, nitric oxide producing hydrogels, PPAR agonist such as PPAR-α gands, tissue plasminogen activator, statins such as atorvastatin, erythropoietin, darbepotin, serine proteinase-1 (SERP-1) and pravastatin, steroids, and/or antibiotics.

Pharmaceutical compositions may be incorporated into the polymers or may be coated on the surface of the polymers after mixing and extrusion by spraying, dipping or painting or microencapsulated and then blended into the polymer mixture. U.S. Patent No. 6,020,385. If the pharmaceutical compositions are covalently bound to the polymer blend, they may be linked by hetero- or homo-bifunctional cross linking agents (see, http://www.piercenet.com/products/browse.cfm?fldID=020306).

Pharmaceutical agents that may be incorporated into the scaffolds or may be coated on the scaffolds include, but are not limited to, (i) pharmacological agents such as, (a) antithrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, thymidine kinase inhibitors, rapamycin, 40-0-(2- Hydroxyethyl)rapamycin (everolimus), 40-0-Benzyl-rapamycin, 40-0(4'-Hydroxymethyl)benzyl-rapamycin, 40-0-[4'-(1,2-Dihydroxyethyl)]benzyl-rapamycin, 40-Allyl-rapamycin, 40-0-[3'-(2,2-Dimethyl-1,3-dioxolan-4(S)-yl-prop-2'-en-1'-yl]-20 rapamycin, (2':E,4'S)-40-0-(4',5'.:Dihydroxypent-2'-en-1-yl),r apamycin 40-0(2Hydroxy) ethoxycar-bonylmethyl-rapamycin, 40-0-(3-Hydroxypropyl-rapamycin 40-0-((Hydroxy)hexyl-rapamycin 40-0-[2-(2-Hydroxy)ethoxy]ethyl-rapamycin, 40-0-[(3S)-2,2Dimethyldioxolan-3-yl]methyl-rapamycin, 40-0-[(2S)-2,3-Dihydroxyprop-1-yl]-rapamycin, 40-0-(2-Acctoxy)ethyl-rapamycin, 40-0-(2-Nicotinoyloxy)ethyl-rapamycin, 40-0-[2-(N-25 Morpholino)acetoxyethyl-rapamycin, 40-0-(2-N-Imidazolylacetoxy)ethyl-rapamycin, 40-0[2-(N-Methyl-N'-piperazinyl)acetoxy]ethyl-rapamycin, 39-0-Desmethyl-3.9,40-0,0 ethylene-rapamycin, (26R)-26- Dihydro-40-0-(2-hydroxy)ethyl-rapamycin, 28-O Methyrapamycin, 40-0-(2-Aminoethyl)-rapamycin, 40-0-(2-Acetaminoethyl)-rapamycin 40-0(2-Nicotinamidoethyl)-rapamycin, 40-0-(2-(N-Methyl- imidazo- 2' ylcarbcthoxamido)ethyl)- 30 rapamycin, 40-0-(2-Ethoxycarbonylaminoethyl)-rapamycin, 40-0-(2-Tolylsulfonamidoethyl)- rapamycin, 40-0-[2-(4',5'-Dicarboethoxy-1',2';3'-triazol-1'-yl)-ethyl]rapamycin, 42-Epi-(telrazolyl)rapamycin (tacrolimus), and 42-[3-hydroxy-2-(hydroxymethyl)-2-methylpropanoate]rapamycin (temsirolimus) (WO2008/086369); (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e. g. , tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; and, (o) agents that interfere with endogenous vasoactive mechanisms, (ii) genetic therapeutic agents include anti-sense DNA and RNA as well as DNA coding for (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor a and P, platelet-derived endothelial growth factor, platelet-derived growth factor, tumor necrosis factor a, hepatocyte growth factor and insulin-like growth factor, (d) cell cycle inhibitors including CD inhibitors, and (e) thymidine kinase ("TK") and other agents useful for interfering with cell proliferation.

Other pharmaceutical agents that may be incorporated into the scaffolds include, but are not limited to, acarbosc, antigens, beta-receptor blockers, non-steroidal antiinflammatory drugs (NSAID, cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomcthasone, betahistine, bezafibrate, bicalutamide, diazepam and diazepam derivatives, budesonide, bufexamac, buprcnorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidinc, cotrimoxazole, codeine, caffeine, vitamin D and derivatives of vitamin D, colestyramine, cromoglicic acid, coumarin and coumarin derivatives, cysteine, cytarabine, cyclophosphamide, cyclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, erythropoietin and erythropoietin derivatives, morphinans, calcium antagonists, irinotecan, modafmil, orlistat, peptide antibiotics, phenytoin, riluzoles, risedronate, sildenafil, topiramatc, macrolide antibiotics, oestrogen and oestrogen derivatives, progestogen and progestogen derivatives, testosterone and testosterone derivatives, androgen and androgen derivatives, ethenzamide, etofenamate, ctofibrate, fcnofibrate, etofylHne, etoposide, famciclovir, famotidine, felodipine, fenoftbrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mcpindolol, meprobamate, meropenem, mesalazinc, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastinc, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tctracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazolinc, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobutcrol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorclbinc, vinpocetine, viquidil, warfarin, xantinol nicotinate, xipamide, zafirlukast, zalcitabine, zidovudine, zolmitriptan, Zolpidem, zoplicone, zotipine and the like. See, e.g., U.S. Patent Nos. 6,897,205, 6,838,528 and 6,497,729.

The stent may also be coated with at least one type of antibodies. For example, the stent may be coated with antibodies or polymeric matrices which are capable of capturing circulating endothelial cells. U.S. Patent No. 7,037,772 (see also, U.S. Patent Publications Nos. 20070213801, 200701196422, 20070191932, 20070156232, 20070141107, 20070055367, 20070042017,20060135476,20060121012).

The scaffold of the present invention may also be formed from metal such as nickel-titanium (Ni-Ti). A metal composition and process of manufacturing the device is disclosed in U.S. Patent No. 6,013,854. The super elastic metal for the device is preferably a super elastic alloy. A super elastic alloy is generally called "a shape-memory alloy" and resumes its original shape after being deformed to such a degree that an ordinary metal undergoes permanent deformation. Super elastic alloys useful in the invention include: Elgiloy® and Phynox® spring alloys (Elgiloy® alloy is available from Carpenter Technology Corporation of Reading Pa.; Phynox® alloy is available from Metal Imphy of Imphy, France), 316 stainless steel and MP35N alloy which are available from Carpenter Technology corporation and Latrobe Steel Company of Latrobe, Pa., and superelastic Nitinol nickel-titanium alloy which is available from Shape Memory Applications of Santa Clara, Calif. U.S. Patent No. 5,891,191.

The scaffold of the present invention may be manufactured in numerous ways. The device may be formed from a tube by removing various portions of the tube's wall to form the patterns described herein. The resulting device will thus be formed from a single contiguous piece of material, eliminating the need for connecting various segments together. Material from the tube wall may be removed using various techniques including laser (YAG laser for example), electrical discharge, chemical etching, metal cutting, a combination of these techniques, or other well known techniques. U.S. Patent Nos. 5,879,381 and 6,117,165. Forming stents in this manner allows for creation of a substantially stress-free structure. In particular, the length may be adapted to that of the diseased part of the lumen in which the stent is to be placed. This may avoid using separate stents to cover the total diseased area.

In an alternate embodiment, a method for fabricating a tube-shaped stent comprising: preparing a racemic poly-lactide mixture; fabricating a biodegradable polymer tube of the racemic poly-lactide mixture; laser cutting the tube until such scaffold is formed. In this embodiment, the fabrication of the scaffold can be performed using a molding technique, which is substantially solvent-free, or an extrusion technique.

Reference is also made to U.S. Patent Nos. 7,329,277, 7,169,175, 7,846,197, 7,846,361, 7,833,260, 6,0254,688, 6,254,631, 6,132,461, 6,821,292, 6,245,103 and 7,279,005. In addition, U.S. Patent Application Nos. 11/781,230, 12/507,663, 12/508,442, 12/986,862, 11/781,233, 12/434,596, 11/875,887, 12/464,042, 12/576,965, 12/578,432, 11/875,892, 11/781,229, 11/781,353, 11/781,232, 11/781,234, 12/603,279, 12/779,767 and 11/454,968, as well as U.S. Patent Publication No. 2001/0029397.

The scope of the present invention is not limited by what has been specifically shown and described hereinabove but only by the language of the claims. Those skilled in the art will recognize that there are suitable alternatives to the depicted examples of materials, configurations, constructions and dimensions. Numerous references, including patents and various publications, are cited and discussed in the description of this invention. The citation and discussion of such references is provided merely to clarify the description of the present invention and is not an admission that any reference is prior art to the invention described herein. While certain embodiments of the present invention have been shown and described, it will be obvious to those skilled in the art that changes and modifications may be made without departing from the scope of the invention as long as covered by the language of the claims. The matter set forth in the foregoing description and accompanying drawings is offered by way of illustration only and not as a limitation.

## Claims

1. A scaffold of a bio-degradable composition, comprising a primarily cylindrically shaped main body with a cylindrical axis having a plurality of circumferential elements (5-12) that circumscribe a circumference of said cylindrical main body, wherein a circumferential element (5,7,9,11) is connected to an adjacent circumferential element (6,8,10,12) by a plurality of first connection elements (13-18) to form a pair (1-4) of circumferential elements, wherein a pair of circumferential elements is connected to an adjacent pair of circumferential elements by a plurality of second connection elements (19-21), wherein the circumferential elements comprise a plurality of undulations, having alternating peaks and valleys, and wherein first and second connection elements are connected with one another to form at least one contiguous spiral pattern, when the scaffold is expanded, **characterized in that** said first connections elements (217,217',401,404-409) are S-shaped or Z-shaped and connect each valley of the undulations in one circumferential element (5,7,9,11) within a pair (1-4) to each peak of the undulations in the other circumferential element (6,8,10,12) of that pair (1-4), and **in that** said second connection elements (19-21) are S-shaped or Z-shaped and connect every other peak of the undulations in one circumferential element (6,8,10) within a pair to a valley of the undulations in an adjacent circumferential element (7,9,11) of an adjacent pair of circumferential elements.

2. The scaffold according to claim 1 or 2, **characterized in that** the undulations form a sinusoidal pattern.

3. The scaffold according to claim 2, **characterized in that** the sinusoidal pattern of one circumferential element in the pair is 180 degrees out-of-phase with the sinusoidal pattern of the other circumferential element in the pair.

4. The scaffold according to claim 2 or 3, **characterized in that** the sinusoidal patterns of one pair of circumferential elements is in-phase with the sinusoidal pattern of an adjacent pair of circumferential elements.

5. The scaffold according to any of the preceding claims, **characterized in that** there are three first connection elements and there are three second connection elements.

6. The scaffold according to any of the preceding claims, **characterized in that** at least one first connection element contains a marker dot.

7. The scaffold according to claim 1, **characterized in that** the spiral pattern forms a helix.

8. The scaffold according to claim 7 **characterized in that** there are two helices substantially parallel to each other.

9. The scaffold according to any of the preceding claims, **characterized in that** the scaffold comprises bioabsorbable polymeric material, particularly poly-L-lactide (PLLA), or biocorrodable metal, particularly iron or magnesium.

10. The scaffold according to any of the preceding claims, **characterized in that** the spiral pattern is formed from a repeated unit comprising, the first connection element and second connection element.

## Patentansprüche

1. Gerüst einer bioabbaubaren Verbindung, umfassend einen vorwiegend zylindrischen Hauptkörper mit einer Zylinderachse und einer Mehrzahl von Umfangselementen (5-10), einen Umfang des zylindrischen Hauptkörpers umschreibend; ein Umfangselement (5, 7, 9, 11) ist an ein benachbartes Umfangselement (6, 8, 10, 12) durch eine Mehrzahl erster Verbindungselemente (13-18) angeschlossen, um ein Paar (1-4) Umfangselemente zu bilden; ein Paar Umfangselemente ist angeschlossen an ein benachbartes Paar von Umfangselementen durch eine Mehrzahl zweiter Verbindungselemente (19-21); die Umfangselemente umfassen eine Mehrzahl von Wellen mit abwechselnden Gipfel und Täler; dabei sind erste und zweite Verbindungselemente miteinander verbunden, um wenigstens ein kontinuierliches Spiralmuster zu bilden, wenn das Gerüst expandiert ist, **dadurch gekennzeichnet, dass** die ersten Verbindungselemente (217, 217', 401, 404-409) S-förmig oder Z-förmig sind und jedes Tal der Wellen in einem Umfangselement (5, 7, 9, 11) mit einem Paar (1-4) mit jedem Gipfel der Wellen im anderen Umfangselement (6, 8, 10, 12) des Paares (1-4) verbinden, und dass die zweiten Verbindungselemente (19-21) S-förmig oder Z-förmig sind und jeden weiteren Gipfel der Wellen in einem Umfangselement (6, 8, 10) innerhalb eines Paares mit einem Tal der Wellen in einem benachbarten Umfangselement (7, 9, 11) eines benachbarten Paares von Umfangselementen verbindet.

2. Gerüst gemäß Anspruch 1, **dadurch gekennzeichnet, dass** die Wellen ein sinusförmiges Muster bilden.

3. Gerüst nach Anspruch 2, **dadurch gekennzeichnet, dass** das sinusförmige Muster eines Umfangselementes in einem Paar um 180° zu dem sinusförmigen Muster des anderen Umfangselementes im Paar verschoben ist.

4. Gerüst nach einem der Ansprüche 2 oder 3, **dadurch gekennzeichnet, dass** die sinusförmigen Muster eines Paares von Umfangselementen mit dem sinusförmigen Muster eines benachbarten Paares von Umfangselementen phasengleich ist.

5. Gerüst gemäß einem der vorausgegangenen Ansprüche, **dadurch gekennzeichnet, dass** drei erste und drei zweite Verbindungselemente vorgesehen sind.

6. Gerüst gemäß einem der vorausgegangenen Ansprüche, **dadurch gekennzeichnet, dass** wenigstens ein Verbindungselement einen Markierpunkt umfasst.

7. Gerüst nach Anspruch 1, **dadurch gekennzeichnet, dass** das Spiralmuster ein Schraubenmuster ist.

8. Gerüst nach Anspruch 7, **dadurch gekennzeichnet, dass** zwei Schraubenmuster vorgesehen sind, im Wesentlichen parallel zueinander verlaufend.

9. Gerüst nach einem der vorausgegangenen Ansprüche, **dadurch gekennzeichnet, dass** das Gerüst ein bioabsorbierbares polymeres Material umfasst, insbesondere ein Poly-L-Lactid (PLLA) oder biokorrodierbares Material, insbesondere Eisen oder Magnesium.

10. Gerüst nach einem der vorausgegangenen Ansprüche, **dadurch gekennzeichnet, dass** das Spiralmuster gebildet ist aus einer sich wiederholenden Einheit, umfassend das erste und das zweite Verbindungselement.

## Revendications

1. Un échafaudage dont le composition est biodégradable, comprenant un corps principal de forme essentiellement cylindrique avec un axe cylindrique possédant une pluralité d'éléments circonférentiels (5-12) qui délimitent une circonférence dudit corps cylindrique principal, un élément circonférentiel (5, 7, 9, 11) étant raccordé à un élément circonférentiel adjacent (6, 8, 10, 12) par une pluralité de premiers éléments de raccordement (13-18) pour former une paire (1-4) d'éléments circonférentiels, une paire d'éléments circonférentiels étant raccordée à une paire adjacente d'éléments circonférentiels par une pluralité de seconds éléments de raccord (19-21), les éléments circonférentiels comprenant une pluralité d'ondulations, présentant une alternance de pointes et de creux, et les premiers et seconds éléments de raccord étant raccordés les uns aux autres pour former au moins un motif en spirale contigu, lorsque l'échafaudage est déployé, **se caractérisant en ce que** lesdits premiers éléments de raccord (217, 217', 401, 404-409) sont en forme de S ou de Z et raccordent chaque creux des ondulations dans un élément circonférentiel (5, 7, 9, 11) au sein d'une paire (1-4) à chaque pointe des ondulations dans les autres éléments circonférentiels (6, 8, 10, 12) de cette paire (1-4), et **en ce que** lesdits seconds éléments de raccord (19-21) sont en forme de S ou de Z et raccordent une pointe sur deux des ondulations en un élément circonférentiel (6, 8, 10) au sein d'une paire à un creux des ondulations dans un élément circonférentiel adjacent (7, 9, 11) d'une paire adjacente d'éléments circonférentiels.

2. L'échafaudage selon la revendication 1 ou 2, **se caractérisant en ce que** les ondulations forment un motif sinusoïdal.

3. L'échafaudage selon la revendication 2, **se caractérisant en ce que** le motif sinusoïdal d'un élément circonférentiel dans la paire est déphasé de 180 degrés par rapport au motif sinusoïdal de l'autre élément circonférentiel dans la paire.

4. L'échafaudage selon la revendication 2 ou 3, **se caractérisant en ce que** les motifs sinusoïdaux d'une paire d'éléments circonférentiels sont en phase avec le motif sinusoïdal d'une paire adjacente d'éléments circonférentiels.

5. L'échafaudage selon l'une quelconque des revendications précédentes, **se caractérisant en ce qu'**il y a trois premiers éléments de raccord et **en ce qu'**il y a trois seconds éléments de raccord.

6. L'échafaudage selon l'une quelconque des revendications précédentes, **se caractérisant en ce qu'**au moins un premier élément de raccord contient un point de repère.

7. L'échafaudage selon la revendication 1, **se caractérisant en ce que** le motif en spirale forme une hélice.

8. L'échafaudage selon la revendication 7 **se caractérisant en ce qu'**il y a deux hélices substantiellement parallèles l'une à l'autre.

9. L'échafaudage selon l'une quelconque des revendications précédentes, **se caractérisant en ce que** l'échafaudage comprend du matériel polymérique bioabsorbable, en particulier du poly-L-lactide (PLLA), ou du métal bio-corrodable, en particulier de l'acier ou du magnésium.

10. L'échafaudage selon l'une quelconque des revendications précédentes, **se caractérisant en ce que** le motif en spirale est constitué d'une unité répétée comprenant le premier élément de raccord et le second élément de raccord.
